# EUROPEAN PATENT APPLICATION

(11) **EP 4 516 791 A1**
(43) Date of publication of application: **05.03.2025**
(21) Application number: 23795606.5
(22) Date of filing: 28.04.2023
(51) Int. Cl.: C07D 471/04, C07D 487/04, C07D 405/04, A61K 31/496, A61P 35/00

(54) **DEUBIQUITINASE INHIBITOR AND USE THEREOF**

(30) Priority: 28.04.2022 CN 202210471474
(71) Applicant: Chaser Therapeutics, Inc., Hangzhou, Zhejiang 310018 (CN)
(72) Inventor: JIANG, Kun, Hangzhou, Zhejiang 310018 (CN); SHEN, Xiang, Hangzhou, Zhejiang 310018 (CN); XI, Xiaomei, Hangzhou, Zhejiang 310018 (CN)
(74) Representative: Gille Hrabal Partnerschaftsgesellschaft mbB Patentanwälte
(86) International application number: PCT/CN2023/091484
(87) International publication number: WO 2023/208174

(57) **Abstract**

The present invention relates to a compound represented by formula (**I**) or a racemate, a stereoisomer, a tautomer, an isotope-labeled form, an N-oxide, a solvate, a polymorph, a metabolite, an ester, a pharmaceutically acceptable salt or a prodrug thereof, a pharmaceutical composition comprising same, and pharmaceutical use thereof. The compound has the activity of inhibiting USP28 and/or USP25, and has potential pharmaceutical use as a medicament for treating a related disease such as a cancer, an inflammation, an autoimmune disease, a virus infection, and a bacterial infection. The structure represented by the formula **I** is as follows:

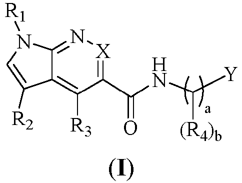

## Description

The present disclosure claims priority to a prior application with the application No. 202210471474.9 and entitled "DEUBIQUITINASE INHIBITOR AND USE THEREOF" filed on China National Intellectual Property Administration on April 28, 2022, which is incorporated herein by reference in its entirety.

### TECHNICAL FIELD

The present disclosure belongs to the field of pharmaceuticals, and particularly relates to a deubiquitinase inhibitor and use thereof.

### BACKGROUND

The normal functioning of a cell depends on intracellular protein homeostasis, and the maintenance of this homeostasis depends on the dynamic balance of protein synthesis and degradation. Protein ubiquitin labeling by ubiquitinase is a major pathway of proteasome degradation, while protein deubiquitination, which is produced by deubiquitinase, is a necessary complement to precise regulation of protein homeostasis. USP25 and USP28 are deubiquitinases whose genes are very close but located in different regions of a cell, and both are closely related to the occurrence and development of various tumor diseases. USP25 and USP28 maintain protein homeostasis by deubiquitinating various oncoproteins, epigenetic drivers, immunoregulatory proteins, and other cytokines. Inhibition of the deubiquitinating activity of USP25 and USP28 can cause excessive degradation of the target protein, ultimately leading to cell death. Therefore, small molecule compounds having inhibitory activity for USP25 and USP28 have potential medical use as a medicament for the treatment of related diseases such as cancer, inflammation, autoimmune diseases, viral infection, and bacterial infection.

### SUMMARY

The present disclosure provides a compound represented by formula **I** below, and a racemate, a stereoisomer, a tautomer, an isotope-labeled form, a *N*-oxide, a solvate, a polymorph, a metabolite, an ester, a pharmaceutically acceptable salt or a prodrug thereof: 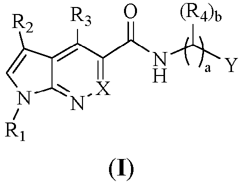 wherein:
X is selected from N or CH;
Y is selected from 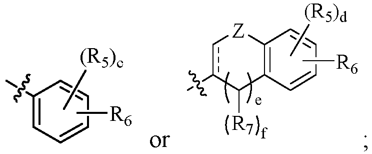
Z is selected from NR₈, O, S, or CR₉R₁₀; the dashed bond represents that there may be a bond or not;
a is selected from 0, 1, 2, 3, 4, 5, or 6;
b is selected from 1 or 2;
c is selected from 1, 2, 3, or 4;
d is selected from 1, 2, or 3;
e is selected from 0 or 1;
f is selected from 1 or 2;
R₁ is selected from hydrogen or optionally unsubstituted or substituted (C₁-C₁₂) aliphatic hydrocarbyl;
R₂ is selected from hydrogen, halogen, or optionally unsubstituted or substituted (C₁-C₁₂) aliphatic hydrocarbyl;
R₃ is selected from halogen, hydroxyl, optionally unsubstituted or substituted (C₁-C₁₂) aliphatic hydrocarbyloxy, or optionally unsubstituted or substituted (C₁-C₁₂) aliphatic hydrocarbylamino;
R₄ may each be identical or different, and are each independently selected from hydrogen, halogen, and optionally unsubstituted or substituted (C₁-C₁₂) aliphatic hydrocarbyl;
R₅ and R₇ may each be identical or different, and are each independently selected from hydrogen, halogen, hydroxyl, amino, optionally unsubstituted or substituted (C₁-C₁₂) aliphatic hydrocarbyl, and optionally unsubstituted or substituted (C₁-C₁₂) aliphatic hydrocarbyloxy;
R₆ is selected from hydrogen, halogen, hydroxyl, amino, and optionally unsubstituted or substituted (C₁-C₁₂) aliphatic hydrocarbyl, or 3- to 20-membered heterocyclyl or 5- to 20-membered heteroaryl containing one, two, or more N atoms and/or O atoms, unsubstituted or optionally substituted with one, two, or more R₁₁;
R₈, R₉, and R₁₀ are selected from hydrogen or optionally unsubstituted or substituted (C₁-C₁₂) aliphatic hydrocarbyl;
R₁₁ may each be identical or different, and are each independently selected from hydrogen, halogen, hydroxyl, amino, and optionally unsubstituted or substituted (C₁-C₁₂) aliphatic hydrocarbyl.

According to an embodiment of the present disclosure, the "unsubstituted (C₁-C₁₂) aliphatic hydrocarbyl" is linear or branched, saturated or unsaturated chain or cyclic hydrocarbyl consisting of 1-12 carbon atoms and corresponding hydrogen atoms, and the type of the aliphatic hydrocarbyl may be selected from alkyl, alkenyl, alkynyl, and other groups; the "substituted (C₁-C₁₂) aliphatic hydrocarbyl" is (C₁-C₁₂) aliphatic hydrocarbyl containing one, two, or more halogen and/or oxygen, sulfur, nitrogen and phosphorus atoms, wherein the halogen and the oxygen, sulfur, nitrogen and phosphorus atoms may be on the linear or branched chain of the (C₁-C₁₂) aliphatic hydrocarbyl or any one position of the linear or branched chain; the "(C₁-C₁₂) aliphatic hydrocarbyl" may preferably be "(C₁-C₁₀) aliphatic hydrocarbyl", "(C₁-C₈) aliphatic hydrocarbyl", and "(C₁-C₆) aliphatic hydrocarbyl", for example, may be selected from the following groups: (C₁-C₆) aliphatic hydrocarbyl, (C₁-C₆) aliphatic hydrocarbyloxy, *N*-(C₁-C₆) aliphatic hydrocarbylamino, *N,*N-di-(C₁-C₃) aliphatic hydrocarbylamino, (C₁-C₆) aliphatic hydrocarbylsulfhydryl, halogenated (C₁-C₆) aliphatic hydrocarbyl, halogenated (C₁-C₆) aliphatic hydrocarbyloxy, (mono- or di-*N*-substituted) halogenated (C₁-C₆) aliphatic hydrocarbylamino, halogenated (C₁-C₆) aliphatic hydrocarbylsulfhydryl, (C₁-C₆) aliphatic hydrocarbyloxy (C₁-C₆) aliphatic hydrocarbyl, (C₁-C₆) aliphatic hydrocarbylsulfhydryl (C₁-C₆) aliphatic hydrocarbyl, *N*-(C₁-C₆) aliphatic hydrocarbylamino (C₁-C₆) aliphatic hydrocarbyl, or *N,N*-di-(C₁-C₃) aliphatic hydrocarbylamino (C₁-C₆) aliphatic hydrocarbyl, and more specifically, may be methyl, fluoromethyl, difluoromethyl, trifluoromethyl, ethyl, propyl, isopropyl, cyclopropyl, butyl, isobutyl, sec-butyl, pentyl and isopentyl, methoxymethyl, ethoxymethyl, propoxymethyl, methoxyethyl, ethoxyethyl, propoxyethyl, methoxypropyl, ethoxypropyl, propoxypropyl, *N-*methylaminomethyl, *N*-methylaminoethyl, *N*-ethylaminoethyl, *N*,*N*-dimethylaminomethyl, *N,N-*dimethylaminoethyl or *N,N-*diethylaminoethyl, cyano, cyanomethyl, cyanoethyl, cyanopropyl, cyanobutyl, or cyanopentyl.

According to an embodiment of the present disclosure, X is selected from CH.

According to an embodiment of the present disclosure, Y is selected from 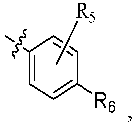 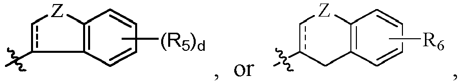 wherein Z, R₅, R₆, and d independently have the definitions described above; for example, Y is selected from

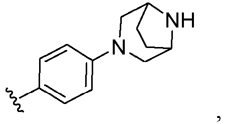

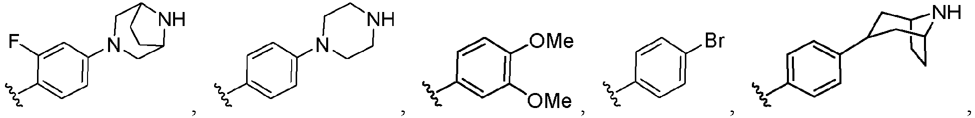

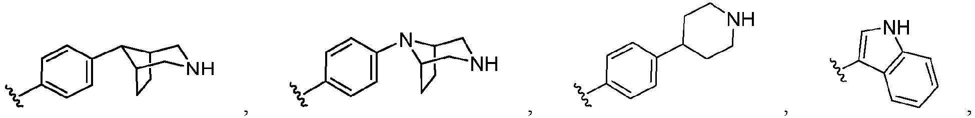

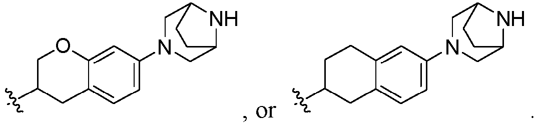

According to an embodiment of the present disclosure, Z is selected from nitrogen-hydrogen (NH), oxygen (O), sulfur (S), or methylene (CH₂).

According to an embodiment of the present disclosure, R₁ is selected from hydrogen or unsubstituted or substituted (C₁-C₆) aliphatic hydrocarbyl; for example, H, (C₁-C₆) aliphatic hydrocarbyl, or halogenated (C₁-C₆) aliphatic hydrocarbyl.

According to an embodiment of the present disclosure, R₁ is selected from hydrogen or optionally unsubstituted or substituted (C₁-C₆) alkyl; for example, H, (C₁-C₆) alkyl, or halogenated (C₁-C₆) alkyl. According to an embodiment of the present disclosure, R₁ is selected from the following groups: H, methyl, ethyl, propyl, isopropyl, butyl, isobutyl, *sec*-butyl, pentyl, isopentyl, *sec*-pentyl, CF₃, CHF₂CH, CH₂FCH, CF₃CH₂, CHF₂CH₂, or CH₂FCH₂.

According to an embodiment of the present disclosure, R₂ is selected from hydrogen or optionally unsubstituted or substituted (C₁-C₆) alkyl; for example, H, (C₁-C₆) alkyl, or halogenated (C₁-C₆) alkyl. According to an embodiment of the present disclosure, R₃ is selected from halogen, hydroxyl, unsubstituted or substituted (C₁-C₆) aliphatic hydrocarbylamino, or unsubstituted or substituted (C₁-C₆) aliphatic hydrocarbyloxy; for example, halogen, (C₁-C₆) hydrocarbylamino, halogenated (C₁-C₆) hydrocarbylamino, (C₁-C₆) hydrocarbyloxy, or halogenated (C₁-C₆) hydrocarbyloxy.

According to an embodiment of the present disclosure, R₃ is selected from halogen, hydroxyl, optionally unsubstituted or substituted (C₁-C₆) alkyloxy, or optionally unsubstituted or substituted (C₁-C₆) alkylamino; for example, F, Cl, Br, I, (C₁-C₆) alkylamino, halogenated (C₁-C₆) alkylamino, (C₁-C₆) alkyloxy, or halogenated (C₁-C₆) alkyloxy.

According to an embodiment of the present disclosure, R₃ is selected from the following groups: Cl, methylamino (Me-NH), fluoromethylamino (CH₂F-NH), difluoromethylamino (CHF₂-NH), trifluoromethylamino (CF₃-NH), ethylamino (Et-NH), propylamino (*ⁿ*Pr-NH), isopropylamino (*ⁱ*Pr-NH), or methoxy (CH₃O).

According to an embodiment of the present disclosure, R₄ is selected from hydrogen or optionally unsubstituted or substituted (C₁-C₆) alkyl; for example, H, (C₁-C₆) alkyl, or halogenated (C₁-C₆) alkyl. According to an embodiment of the present disclosure, R₄ is selected from the following groups: H or methyl.

According to an embodiment of the present disclosure, R₅ is selected from hydrogen, halogen, optionally unsubstituted or substituted (C₁-C₆) alkyl, or optionally unsubstituted or substituted (C₁-C₆) alkoxy; for example, H, F, Cl, Br, I, (C₁-C₆) alkyl, or (C₁-C₆) alkoxy.

According to an embodiment of the present disclosure, R₅ is selected from the following groups: H, F, Br, methyl, or methoxy.

According to an embodiment of the present disclosure, R₆ is selected from hydrogen, halogen, hydroxyl, amino, and optionally unsubstituted or substituted (C₁-C₆) aliphatic hydrocarbyl, or 3- to 14-membered heterocyclyl or 5- to 14-membered heteroaryl containing one, two, or more N atoms and/or O atoms, unsubstituted or optionally substituted with one, two, or more R₁₁; R₁₁ are each identical or different, and are each independently selected from hydrogen, halogen, hydroxyl, amino, and optionally unsubstituted or substituted (C₁-C₆) aliphatic hydrocarbyl.

According to an embodiment of the present disclosure, R₆ is selected from 3- to 10-membered heterocyclyl containing one or two N as heteroatoms, unsubstituted or optionally substituted with one, two, or more R₁₁; R₁₁ are each identical or different, and are each independently selected from hydrogen or (C₁-C₆) alkyl.

According to an embodiment of the present disclosure, R₆ is selected from 6- to 8-membered heterocyclyl containing one or two N atoms and/or O atoms; for example, the following groups:

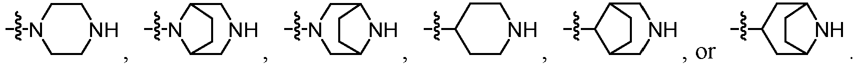

According to an embodiment of the present disclosure, R₇, R₈, R₉, and R₁₀ are identical or different, and are each independently selected from hydrogen or optionally unsubstituted or substituted (C₁-C₆) alkyl; for example, H, (C₁-C₆) alkyl, or halogenated (C₁-C₆) alkyl.

According to an embodiment of the present disclosure, R₇, R₈, R₉, and R₁₀ are selected from the following groups: H or methyl.

According to an embodiment of the present disclosure, the structure of formula **I** is further selected from structures of formula **II-a** and formula **II-b:** 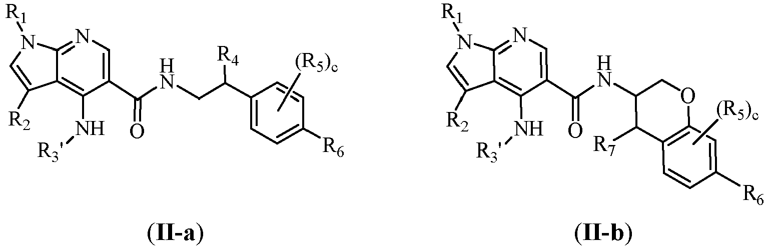 R₁, R₂, R₄, R₅, R₆, R₇, and c described in formula **II-a** and formula **II-b** are as defined for those in formula **I**; R₃' is selected from (C₁-C₆) aliphatic hydrocarbyl or (C₁-C₆) aliphatic hydrocarbyl comprising one, two, or more halogen and/or hydroxyl substitutions. Further, R₃' is preferably methyl, fluoromethyl, difluoromethyl, trifluoromethyl, ethyl, propyl, and isopropyl.

According to an embodiment of the present disclosure, the following compounds (**I-01** to **I-33**), or tautomers, optical isomers, isotope-labeled forms, N-oxides, solvates, pharmaceutically acceptable salts or prodrugs thereof are preferred:

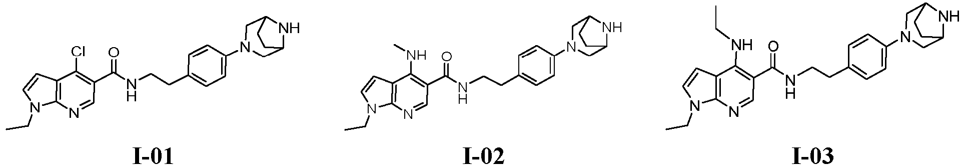

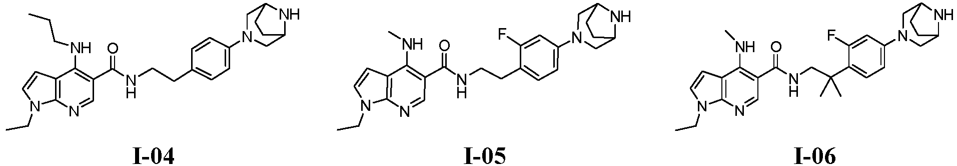

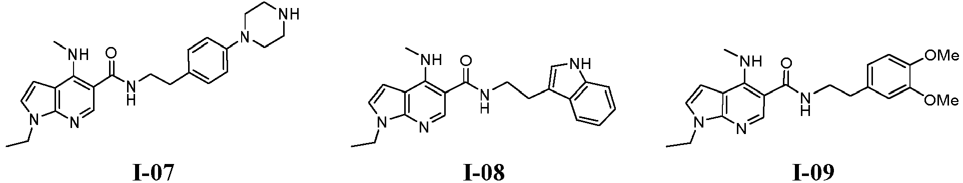

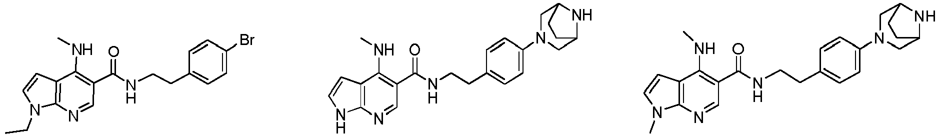

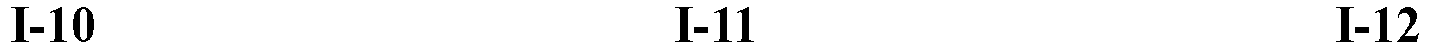

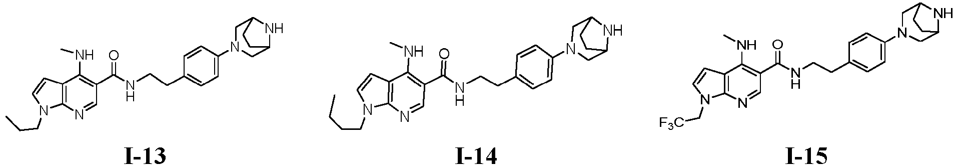

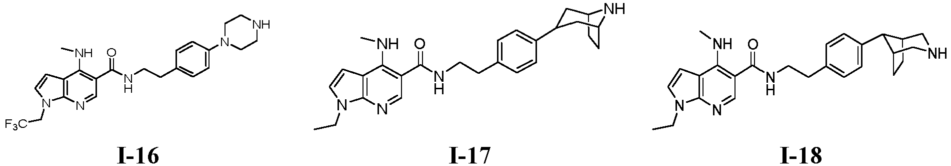

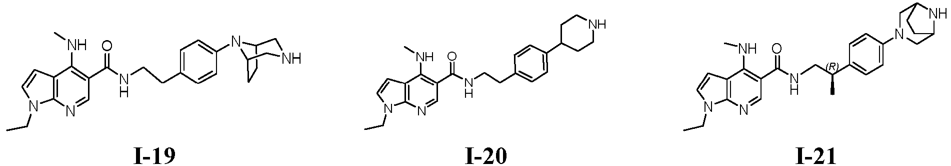

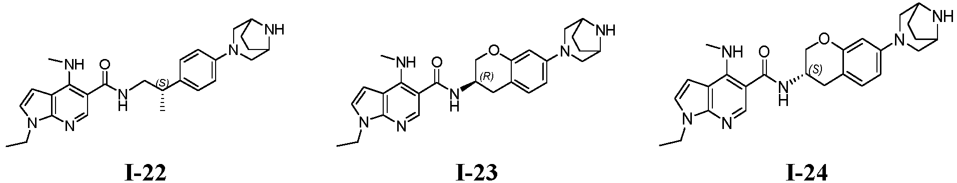

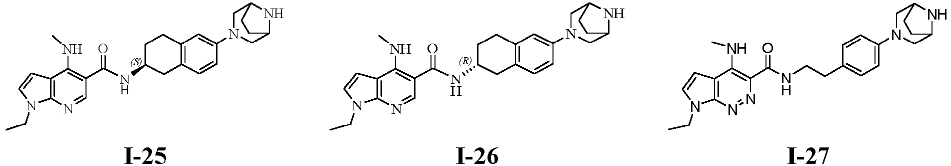

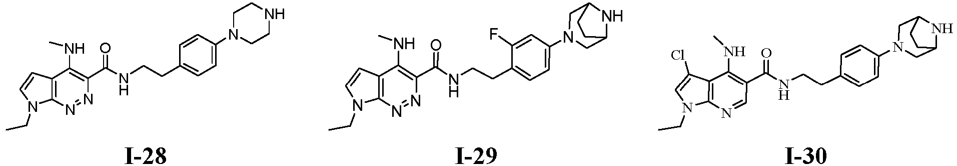

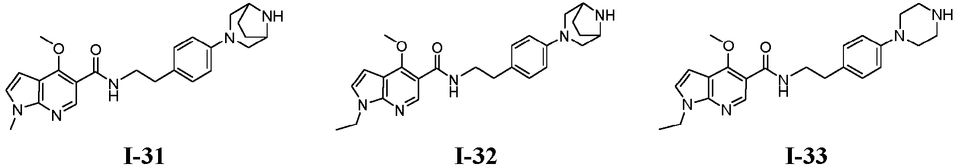

The present disclosure further provides a pharmaceutical composition comprising a compound of formula (**I**), or a racemate, a stereoisomer, a tautomer, an isotope-labeled form, a *N*-oxide, a solvate, a polymorph, a metabolite, an ester, a pharmaceutically acceptable salt or a prodrug thereof as an active ingredient.

According to an embodiment of the present disclosure, the pharmaceutical composition further comprises a therapeutically effective amount of the compound of formula (**I**), or a tautomer, an optical isomer, a N-oxide, a solvate, a pharmaceutically acceptable salt or a prodrug thereof, and a pharmaceutically acceptable carrier.

The carrier in the pharmaceutical composition is "acceptable" in that it is compatible with (and preferably, capable of stabilizing) the active ingredient of the composition and is not deleterious to the subject being treated. One or more solubilizers may be used as pharmaceutical excipients for delivery of the active compound.

The present disclosure further provides use of the compound of formula (**I**), or the racemate, the stereoisomer, the tautomer, the isotope-labeled form, the N-oxide, the solvate, the polymorph, the metabolite, the ester, the pharmaceutically acceptable salt or the prodrug thereof, or the pharmaceutical composition for manufacturing a medicament for the treatment of diseases or disorders related to the inhibition of USP28.

The present disclosure further provides use of the compound of formula (**I**), or the racemate, the stereoisomer, the tautomer, the isotope-labeled form, the *N*-oxide, the solvate, the polymorph, the metabolite, the ester, the pharmaceutically acceptable salt or the prodrug thereof, or the pharmaceutical composition for manufacturing a medicament for the treatment of diseases or disorders related to the inhibition of USP25.

The present disclosure further provides use of the compound of formula (**I**), or the racemate, the stereoisomer, the tautomer, the isotope-labeled form, the *N*-oxide, the solvate, the polymorph, the metabolite, the ester, the pharmaceutically acceptable salt or the prodrug thereof, or the pharmaceutical composition for manufacturing a medicament for the treatment of diseases or disorders related to the inhibition of USP25 and USP28.

The present disclosure further provides a method of treatment or prevention of diseases or disorders related to the modulation of USP28 and/or USP25, which comprises administering to a patient suffering from at least one of the diseases or disorders a compound of formula (**I**), or a racemate, a stereoisomer, a tautomer, an isotope-labeled form, a *N*-oxide, a solvate, a polymorph, a metabolite, an ester, a pharmaceutically acceptable salt or a prodrug thereof.

According to an embodiment of the present disclosure, the diseases or disorders related to USP25 and/or USP28 include cancer, inflammation, autoimmune diseases, viral infection, and bacterial infection.

According to an embodiment of the present disclosure, the pharmaceutical composition may be in a form suitable for oral administration, for example, tablet, troche, lozenge, aqueous or oily suspension, dispersible powder or granule, emulsion, hard or soft capsule, or syrup or elixir. Oral compositions can be prepared according to any method known in the art for preparing pharmaceutical compositions and may comprise one or more ingredients selected from a sweetener, a flavoring agent, a colorant, and a preservative, so as to provide a pleasant-to-eye and palatable pharmaceutical formulation. Tablets contain active ingredients and non-toxic pharmaceutically acceptable excipients which are used for mixing and suitable for the preparation of tablets. These excipients may be inert excipients, granulating agents, disintegrants, adhesives, and lubricants. These tablets may be uncoated or may be coated by known techniques for masking the taste of the drug or delaying the disintegration and absorption of the drug by the gastrointestinal tract and thus enabling sustained release of the drug over a longer period.

According to an embodiment of the present disclosure, the pharmaceutical composition provides an oral formulation in the form of a soft gelatin capsule in which the active ingredient is mixed with an inert solid diluent or in which the active ingredient is mixed with a water-soluble carrier or an oily vehicle. Aqueous suspensions contain active substances and excipients which are used for mixing and suitable for the preparation of aqueous suspensions. Such excipients are suspending agents, dispersants, or wetting agents. These aqueous suspensions may also contain one or more preservatives, one or more colorants, one or more flavoring agents, and one or more sweeteners. Oily suspensions can be prepared by suspending the active ingredient in a vegetable oil, or in a mineral oil. These oil suspensions may contain thickeners. The sweeteners and the flavoring agents described above may be added to provide a palatable formulation. These compositions can be well preserved by the addition of antioxidants; dispersible powders and granules suitable for the preparation of an aqueous suspension can provide an active ingredient, and a dispersant or wetting agent, and a suspending agent or one or more preservatives for mixing by the addition of water. Suitable dispersants or wetting agents and suspending agents may also be added to facilitate the preparation of the formulation as described in the above examples. Other excipients, such as sweeteners, flavoring agents, and colorants, may also be added. These compositions are well preserved by the addition of antioxidants such as ascorbic acid.

According to an embodiment of the present disclosure, the pharmaceutical composition may also be in the form of an oil-in-water emulsion. The oily phase may be a vegetable oil or a mineral oil, or a mixture thereof. Suitable emulsifiers may be naturally occurring phospholipids, and the emulsions may also contain sweeteners, flavoring agents, preservatives, and antioxidants. Such formulations may also contain palliatives, preservatives, colorants, and antioxidants.

According to an embodiment of the present disclosure, the pharmaceutical composition may be in the form of a sterile injectable aqueous solution. Available and acceptable vehicles or solvents include water, Ringer's solution, and isotonic sodium chloride solution. The sterile injectable formulation may be a sterile injectable oil-in-water microemulsion in which the active ingredient is dissolved in the oily phase. The injectable liquid or microemulsion can be locally injected into the bloodstream of a patient in large quantities. Alternatively, it may be desirable to administer solutions and microemulsions in such a way as to maintain a constant circulating concentration of the compound of the present disclosure. To maintain such a constant concentration, a continuous intravenous delivery device may be used. An example of such a device is a Deltec CADD-PLUS.TM.5400 intravenous injection pump.

According to an embodiment of the present disclosure, the pharmaceutical composition may be in the form of a sterile injectable aqueous or oily suspension for intramuscular and subcutaneous administration. The suspension can be prepared according to the known art using those suitable dispersants or wetting agents and suspending agents described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids can also be used to prepare injections.

According to an embodiment of the present disclosure, the compound of the present disclosure may be administered in the form of a suppository for rectal administration. These pharmaceutical compositions can be prepared by mixing a drug with a suitable non-irritating excipient which is solid at ordinary temperatures but liquid in the rectum and therefore will melt in the rectum to release the drug.

As is well known to those skilled in the art, the dosage of the drug administered depends on a variety of factors, including, but not limited to, the activity of the specific compound used, the age of the patient, the weight of the patient, the health condition of the patient, the behavior of the patient, the diet of the patient, the time of administration, the mode of administration, the rate of excretion, the combination of drugs, and the like. In addition, the optimal treatment regimen, such as the mode of treatment, the daily amount of the compound of general formula (**I**), or the type of pharmaceutically acceptable salts, can be verified according to conventional treatment regimens.

### Beneficial Effects

The present disclosure provides a deubiquitinase inhibitor compound. The series of compounds have good inhibitory activity for USP25 and/or USP28, and can be used as a medicament for preventing or treating diseases related to deubiquitinase.

### Definitions and Description

Unless otherwise stated, the definitions of groups and terms described in the description and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the description of the present application.

When a numerical range defined by an "integer" is recited in the description and claims of the present application, it shall be construed as reciting both endpoints of the range and every integer within the range. For example, "an integer of 0-6" shall be construed as including every integer of 0, 1, 2, 3, 4, 5, and 6. "More" refers to three or more.

The term "halogen" refers to F, Cl, Br, and I.

The term "aliphatic hydrocarbyl" includes saturated or unsaturated, and linear or branched chain or cyclic hydrocarbyl. The aliphatic hydrocarbyl may be selected from alkyl, alkenyl, alkynyl, and the like, has preferably 1-12 or 1-10 carbon atoms, and more preferably 1-6 carbon atoms, and specifically may include, but is not limited to, the following groups: methyl, ethyl, propyl, isopropyl, *n*-butyl, isobutyl, *tert*-butyl, *n*-pentyl, isopentyl, neopentyl, *n*-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, cyclopropyl, cyclobutyl, cyclopentyl, and cyclohexyl. The aliphatic hydrocarbyl may optionally comprise one or more other suitable substituents. Examples of the substituents described above may include hydroxyl, halogen, cyano, amino, and other groups. For example, the aliphatic hydrocarbyl may contain one, two, or more halogens, which means that one, two, or more hydrogen atoms of the aliphatic hydrocarbyl may be substituted with an equivalent number of halogens. If the hydrocarbyl contains more than one carbon atoms, then those carbons are not necessarily connected to each other. For example, at least two of the carbons may be connected via a suitable atom or group. That is, the aliphatic hydrocarbyl group may optionally contain one, two, or more heteroatoms (or may be construed as optional insertion of heteroatoms into the aliphatic hydrocarbyl at any C-C bond or C-H bond). Suitable heteroatoms will be apparent to those skilled in the art and include, for example, sulfur, nitrogen, oxygen, phosphorus, and silicon. The aliphatic hydrocarbyl group containing heteroatoms may be selected from the following groups: (C₁-C₆) aliphatic hydrocarbyloxy, (C₁-C₆) aliphatic hydrocarbylsulfhydryl, halogenated (C₁-C₆) aliphatic hydrocarbyl, halogenated (C₁-C₆) aliphatic hydrocarbyloxy, halogenated (C₁-C₆) aliphatic hydrocarbylsulfhydryl, (C₁-C₆) aliphatic hydrocarbyloxy (C₁-C₆) aliphatic hydrocarbyl, (C₁-C₆) aliphatic hydrocarbylsulfhydryl (C₁-C₆) aliphatic hydrocarbyl, *N*-(C₁-C₃) aliphatic hydrocarbylamino (C₁-C₆) aliphatic hydrocarbyl, or *N,*N-di-(C₁-C₃) aliphatic hydrocarbylamino (C₁-C₆) aliphatic hydrocarbyl, for example, methoxymethyl, ethoxymethyl, propoxymethyl, methoxyethyl, ethoxyethyl, propoxyethyl, methoxypropyl, ethoxypropyl, propoxypropyl, *N*-methylaminomethyl, *N*-methylaminoethyl, *N*-ethylaminoethyl, *N,N-*dimethylaminomethyl, *N*,*N*-dimethylaminoethyl, *N*,*N*-diethylaminoethyl, cyano, cyanomethyl, cyanoethyl, cyanopropyl, cyanobutyl, or cyanopentyl; the "aliphatic hydrocarbyl" moiety contained in the other groups is as defined above.

The term "3- to 20-membered heterocyclyl" refers to a saturated monovalent monocyclic or bicyclic hydrocarbon ring containing 1-5 heteroatoms independently selected from N, O, and S, preferably "3- to 10-membered heterocyclyl". The term "3- to 10-membered heterocyclyl" refers to a saturated monovalent monocyclic or bicyclic hydrocarbon ring containing 1-5, preferably 1-3, heteroatoms selected from N, O, and S. The heterocyclyl may be attached to the rest of the molecule through any one of the carbon atoms or the nitrogen atom (if present). In particular, the heterocyclyl may include, but is not limited to, 4-membered rings such as azetidinyl and oxetanyl; 5-membered rings such as tetrahydrofuranyl, dioxolyl, pyrrolidinyl, imidazolidinyl, pyrazolidinyl, and pyrrolinyl; 6-membered rings such as tetrahydropyranyl, piperidyl, morpholinyl, dithianyl, thiomorpholinyl, piperazinyl, or trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[*c*]pyrrol-2(1*H*)-yl ring, or a 5,6-membered bicyclic ring such as a hexahydropyrrolo[1,2-*a*]pyrazin-2(1*H*)-yl ring. The ring containing the nitrogen atom may be partially unsaturated, that is, it may contain one or more double bonds, for example, but not limited to, 2,5-dihydro-1*H*-pyrrolyl, 4*H*-[1,3,4]thiadiazinyl, 4,5-dihydrooxazolyl, or 4*H*-[1,4]thiazinyl, or it may be benzo-fused, for example, but not limited to, dihydroisoquinolyl. According to the present disclosure, the heterocyclyl is non-aromatic. The 3- to 10-membered heterocyclyl may be further selected from the following groups:

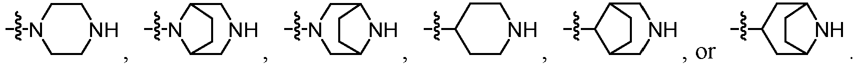

Unless otherwise stated, heterocyclyl or heteroaryl includes all possible isomeric forms thereof, e.g., position isomers thereof. Accordingly, for some illustrative non-limiting examples, pyridinyl or pyridinylene includes pyridin-2-yl, pyridinylene-2-yl, pyridin-3-yl, pyridinylene-3-yl, pyridin-4-yl, and pyridinylene-4-yl; thienyl or thienylene includes thien-2-yl, thien-2-ylene, thien-3-yl, and thien-3-ylene.

In any method for preparing the compound of the present disclosure, it may be necessary and/or desirable to protect sensitive or reactive groups on any molecule concerned. This can be achieved by conventional protective groups, as described in textbooks or in reference books in the art. The protective group may be removed at a convenient subsequent stage using methods known in the art. Those skilled in the art will recognize that, other reagents, including, but not limited to, Pd/C, Pd(OH)₂, PdCl₂, Pd(OAc)₂/Et₃SiH, Raney nickel, an appropriately selected acid, an appropriately selected base, fluoride, and the like, may be used in this deprotection step depending on the specific protective group.

The target compound may be separated according to known methods, for example, by extraction, filtration, column chromatography, FCC, or preparative HPLC.

According to the molecular structure, the compounds of the present disclosure may be chiral and may therefore be present in various enantiomeric forms. These compounds may therefore be present in a racemic or optically active form. The compounds of the present disclosure or intermediates thereof may be separated into enantiomers by chemical or physical methods well known to those skilled in the art, or used in such form for synthesis. In the case of racemic amines, diastereoisomers are prepared from mixtures by reaction with optically active resolving agents. Examples of suitable resolving agents are optically active acids such as *R*- or *S*-tartaric acid, diacetyltartaric acid, dibenzoyltartaric acid, mandelic acid, malic acid, lactic acid, suitable *N*-protected amino acids (e.g., *N*-benzoylproline or *N*-benzenesulfonylproline), or various optically active camphorsulfonic acids. Enantiomeric resolution by chromatography can be advantageously performed with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvents containing water or alcohol, for example, hexane/isopropanol/acetonitrile.

Those skilled in the art will understand that not all nitrogen-containing heterocyclic rings can form *N*-oxides, as nitrogen needs to have available lone pairs of electrons used for oxidation to oxides; those skilled in the art will identify nitrogen-containing heterocyclic rings capable of forming *N-*oxides. Those skilled in the art will also recognize that tertiary amines are capable of forming *N-*oxides. Synthesis methods for preparing *N*-oxides of heterocyclic rings and tertiary amines are well known to those skilled in the art and include oxidation of heterocyclic rings and tertiary amines by peroxy acids such as peroxyacetic acid and *m*-chloroperbenzoic acid, hydrogen peroxide, alkyl hydroperoxides such as *tert*-butyl hydroperoxide, sodium perborate, and dioxiranes such as dimethyldioxirane. These methods for preparing *N*-oxides have been widely described and reviewed in the literature.

A pharmaceutically acceptable salt may be, for example, acid addition salts of the compounds of the present disclosure having a nitrogen atom in the chain or ring and having sufficient basicity, for example, acid addition salts formed with the following inorganic acids: hydrochloric acid, hydrofluoric acid, hydrobromic acid, hydroiodic acid, sulfuric acid, pyrosulfuric acid, phosphoric acid, or nitric acid; hydrosulfates; or acid addition salts formed with the following organic acids: formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxylbenzoyl)benzoic acid, camphoric acid, cinnamic acid, cyclopentanepropionic acid, digluconic acid, 3-hydroxyl-2-naphthoic acid, nicotinic acid, pamoic acid, pectinic acid, peroxosulfuric acid, 3-phenylpropionic acid, picric acid, pivalic acid, 2-hydroxylethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptoic acid, glycerophosphoric acid, aspartic acid, sulfosalicylic acid, hemisulfuric acid, or thiocyanic acid.

In addition, another suitable pharmaceutically acceptable salt of the compounds of the present disclosure having sufficient acidity is an alkali metal salt (e.g., sodium salt or potassium salt), an alkaline earth metal salt (e.g., calcium salt or magnesium salt), an ammonium salt, or a salt formed with an organic base which provides a physiologically acceptable cation, for example, a salt formed with the following substances: a sodium ion, a potassium ion, *N*-methylglucamine, dimethylglucamine, ethylglucamine, lysine, dicyclohexylamine, 1,6-hexanediamine, ethanolamine, glucosamine, meglumine, sarcosine, serinol, trihydroxylmethylaminomethane, aminopropanediol, or 1-amino-2,3,4-butanetriol. As an example, the pharmaceutically acceptable salts include salts formed from the group -COOH with the following substances: a sodium ion, a potassium ion, a calcium ion, a magnesium ion, *N*-methylglucamine, dimethylglucamine, ethylglucamine, lysine, dicyclohexylamine, 1,6-hexanediamine, ethanolamine, glucosamine, meglumine, sarcosine, serinol, trihydroxylmethylaminomethane, aminopropanediol, or 1-amino-2,3,4-butanetriol.

In addition, the basic nitrogen-containing groups may be quaternized with the following agents: lower alkyl halides such as methyl, ethyl, propyl, and butyl chlorides, bromides, and iodides; dialkyl sulfates such as dimethyl sulfate, diethyl sulfate, dibutyl sulfate, and dipentyl sulfate; long chain halides such as decyl, lauryl, myristyl, and stearyl chlorides, bromides, and iodides; and aralkyl halides such as benzyl and phenethyl bromides. As an example, pharmaceutically acceptable salts include hydrochloride, sulfate, nitrate, bisulfate, hydrobromide, acetate, oxalate, citrate, mesylate, formate, meglumine, or the like.

Since the compounds of the present disclosure may have a plurality of salt-forming sites, the "pharmaceutically acceptable salt" includes not only a salt formed at 1 salt-forming site of the compounds of the present disclosure but also salts formed at 2, 3, or all of the salt-forming sites thereof. For this purpose, in the "pharmaceutically acceptable salt", the molar ratio of the compound of formula (**I**) to a radical ion (anion) of an acid or a cation of a base required for salt formation may vary within a wide range, and may be, for example, 4:1-1:4, such as 3:1, 2:1, 1:1, 1:2, or 1:3.

According to the present disclosure, the pharmaceutically acceptable anions include anions selected from those generated by the ionization of inorganic or organic acids. The "inorganic acid" includes, but is not limited to, hydrochloric acid, hydrofluoric acid, hydrobromic acid, hydroiodic acid, sulfuric acid, pyrosulfuric acid, phosphoric acid, or nitric acid. The "organic acid" includes, but is not limited to, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxylbenzoyl)benzoic acid, camphoric acid, cinnamic acid, cyclopentanepropionic acid, digluconic acid, 3-hydroxyl-2-naphthoic acid, nicotinic acid, pamoic acid, pectinic acid, peroxosulfuric acid, 3-phenylpropionic acid, picric acid, pivalic acid, 2-hydroxylethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptoic acid, glycerophosphoric acid, aspartic acid, sulfosalicylic acid, hemisulfuric acid, or thiocyanic acid.

According to the position and nature of the various substituents, the compounds of the present disclosure may also comprise one or more asymmetric centers. Asymmetric carbon atoms may be present in either the (*R*) or (*S*) configuration. When there is only one asymmetric center, a racemic mixture is generated, and when there are multiple asymmetric centers, a diastereoisomeric mixture is generated. In some cases, asymmetry may also be present due to hindered rotation about a particular bond, for example, the two substituted aromatic rings of a particular compound connected by the central bond may be asymmetric. Furthermore, the substituents may be present in *cis-* or *trans-*isomeric forms.

The compounds of the present disclosure also include all possible stereoisomers thereof, either in the form of a single stereoisomer or in the form of any mixture of the stereoisomers (e.g., *R-* or *S*-isomers, or *E*- or *Z*-isomers) in any proportion. Single stereoisomers (e.g., single enantiomers or single diastereoisomers) of the compounds of the present disclosure may be separated by any suitable method in the prior art (e.g., chromatography, particularly, e.g., chiral chromatography).

The term "tautomer" refers to functional isomers resulting from the rapid movement of an atom in a molecule between two positions. The compounds of the present disclosure may exhibit the tautomerism. A tautomeric compound may be present in two or more interconvertible forms. Prototropic tautomers result from the migration of a covalently bonded hydrogen atom between two atoms. Tautomers are generally present in an equilibrium form. Efforts to separate a single tautomer usually lead to a mixture, the physicochemical properties of which are consistent with those of the mixture of the compound. The position of the equilibrium depends on the chemical properties of the molecule. For example, in many aliphatic aldehydes and ketones such as acetaldehyde, the keto form predominates; whereas in phenols, the enol form predominates. In the present disclosure, all tautomeric forms of the compounds are included.

In the present disclosure, the compounds involved also include isotope-labeled forms, which are identical to the compounds represented by formula (**I**), but have one or more atoms substituted with atoms with the atomic mass or mass number different from the atomic mass or mass number of those usually found in nature. Examples of isotopes that can be incorporated into the compounds of the present disclosure include isotopes of H, C, N, O, S, F, and Cl, such as ²H, ³H, ¹³C, ¹¹C, ¹⁴C, ¹⁵N, ¹⁸O, ¹⁷O, ³²P, ³⁵S, ¹⁸F, and ³⁶Cl. The compounds or the prodrugs thereof, or the pharmaceutically acceptable salts thereof comprising the isotopes described above and/or other isotopes of other atoms are within the scope of the present disclosure. Certain isotope-labeled forms of the present disclosure, e.g., those into which radioactive isotopes such as ³H and ¹⁴C are incorporated, are useful in drug and/or substrate tissue distribution assays. Tritium (i.e., ³H) and carbon 14 (i.e., ¹⁴C) isotopes are particularly preferred due to ease of preparation and detectability. Furthermore, substitution with heavier isotopes such as deuterium (i.e., ²H) may afford certain therapeutic advantages (e.g., increased *in vivo* half-life or reduced dose) resulting from greater metabolic stability and hence may be preferred in some cases. The compounds of the present disclosure as claimed may be particularly limited to substitution with deuterium or tritium. Furthermore, the absence of the term deuterium or tritium when hydrogen is listed as a substituent does not mean that deuterium or tritium is excluded, on the contrary, deuterium or tritium can also be included.

The term "effective amount" or "therapeutically effective amount" refers to an amount of the compounds of the present disclosure sufficient to effect the intended use, including, but not limited to, the treatment of a disease as defined below. The therapeutically effective amount may vary depending on the following factors: the intended use (*in vitro* or *in vivo*), or the subject and diseases or conditions being treated, such as the body weight and age of the subject, severity of the diseases or conditions, and route of administration, which may be readily determined by one of ordinary skill in the art. The specific dosage will vary depending on the following factors: the selected particular compound, the dosage regimen to be followed, whether to administer in combination with other compounds, the schedule of administration, the tissue to be administered, and the physical delivery system carried.

The term "auxiliary material" refers to a pharmaceutically acceptable inert ingredient. Examples of types of excipients include, without limitation, adhesives, disintegrants, lubricants, glidants, stabilizers, fillers, diluents, and the like. Excipients are capable of enhancing the handling characteristics of the pharmaceutical formulation, i.e., making the formulation more amenable to direct compression by increasing flowability and/or adhesiveness. Examples of typical pharmaceutically acceptable carriers suitable for use in the formulations described above include: saccharides, such as lactose, sucrose, mannitol, and sorbitol; starches, such as corn starch, tapioca starch, and potato starch; cellulose and derivatives thereof, such as sodium carboxymethylcellulose, ethyl cellulosem, and methyl cellulose; calcium phosphates, such as dicalcium phosphate and tricalcium phosphate; sodium sulfate; calcium sulfate; polyvinylpyrrolidone; polyvinyl alcohol; stearic acid; alkaline earth metal stearate, such as magnesium stearate and calcium stearate; vegetable oils, such as peanut oil, cottonseed oil, sesame oil, olive oil, and corn oil; nonionic, cationic, and anionic surfactants; a glycol polymer; fatty alcohols; and grain hydrolysis solids and other nontoxic compatible auxiliary materials commonly available in pharmaceutical formulations, such as fillers, adhesives, disintegrants, buffers, preservatives, antioxidants, lubricants, and colorants.

The term "solvate" refers to forms of the compounds of the present disclosure in which a complex is formed by coordination of the compound in a solid or liquid state with solvent molecules. Hydrate is a particular form of solvate in which coordination occurs with water. In the present disclosure, the preferred solvate is a hydrate. Further, pharmaceutically acceptable solvates (hydrates) of the compounds of general formula (**I**) of the present disclosure refer to co-crystals and clathrates formed from the compounds of general formula (**I**) with one or more molecules of water or other solvents in stoichiometric amounts. Available solvents for solvates include, but are not limited to, water, methanol, ethanol, ethylene glycol, and acetic acid.

The term "prodrug", also known as "drug precursor", refers to a compound that is converted *in vivo* to the compound represented by the above general formula or a specific compound. Such conversion is affected by hydrolysis of the prodrug in the blood or by enzymatic conversion of the prodrug into the parent structure in the blood or tissue. The prodrug of the present disclosure may be an ester, and in the present disclosure, the ester that may be used as the prodrug includes phenyl esters, aliphatic (C₁-C₂₄) esters, acyloxymethyl esters, carbonates, carbamates, and amino acid esters. For example, a compound of the present disclosure containing hydroxyl or carboxyl can be acylated to give a prodrug. Other prodrug forms include phosphate esters, and these phosphate esters are obtained by phosphorylation of the hydroxyl on the parent structure.

The "cancer" described herein includes, but is not limited to, bladder cancer, breast cancer (e.g., ductal carcinoma), cervical cancer (e.g., squamous cell carcinoma), colorectal cancer (e.g., adenocarcinoma), esophageal cancer (e.g., squamous cell carcinoma), gastric cancer (e.g., adenocarcinoma, medulloblastoma, colon cancer, choriocarcinoma, squamous cell carcinoma), head and neck cancer, hematologic cancer (e.g., acute lymphocytic anemia, acute myelogenous leukemia, acute lymphocytic leukemia B-cell, anaplastic large cell lymphoma, B-cell lymphoma, Burkitt lymphoma, chronic lymphocytic leukemia, chronic eosinophilic leukemia/hypereosinophilic syndrome, chronic myelogenous leukemia, Hodgkin's lymphoma, mantle cell lymphoma, multiple myeloma, or T-cell acute lymphocytic leukemia), lung cancer (e.g., bronchoalveolar carcinoma, mesothelioma, mucoepidermoid carcinoma, small cell lung cancer, non-small cell lung cancer, adenocarcinoma, or squamous cell carcinoma), liver cancer (e.g., hepatocellular carcinoma), lymphoma, nervous system cancer (e.g., glioblastoma, neuroblastoma, or glioma), ovarian cancer (e.g., adenocarcinoma), pancreatic cancer (e.g., ductal carcinoma), prostate cancer (e.g., adenocarcinoma), kidney cancer (e.g., renal cell carcinoma or renal clear cell carcinoma), sarcoma (e.g., chondrosarcoma, Ewing's sarcoma, fibrosarcoma, multisource sarcoma, osteosarcoma, rhabdomyosarcoma, or synovial sarcoma), skin cancer (e.g., melanoma, epidermoid carcinoma, or squamous cell carcinoma), thyroid cancer (e.g., medullary carcinoma), uterine cancer, and the like.

The "autoimmune disease" or "autoimmune disorder" described herein refers to a condition that is immune-mediated by attack on self-tissues, but may also involve an immune response to a microorganism. Examples of autoimmune diseases include, but are not limited to: multiple sclerosis, psoriasis, inflammatory bowel disease, ulcerative colitis, Crohn's disease, rheumarthritis, multiple arthritis, local and systemic scleroderma, systemic lupus erythematosus, discoid lupus erythematosus, skin erythema, cutaneous lupus erythematosus (including chilblain lupus erythematosus, lupus nephritis, discoid lupus erythematosus, or subacute cutaneous lupus erythematosus, dermatomyositis, polymyositis, idiopathic edema, chronic thyroiditis, Guillain-Barre syndrome, Grave's disease, myasthenia gravis, Sjogren's syndrome, nodular panarteritis, autoimmune enteropathy, uveitis, autoimmune oophoritis, chronic immune thrombocytopenic purpura, colitis, diabetes, psoriasis, pemphigus vulgaris, proliferative glomerulonephritis, Wiscott-Aldrich syndrome, autoimmune lymphoproliferative disorders, chronic arthritis, inflammatory chronic sinusitis, colitis, celiac disease, inflammatory bowel disease, Barlow's esophageal cancer, inflammatory gastritis, autoimmune nephritis, autoimmune vasculitis, autoimmune hepatitis, autoimmune cardioinflammation, autoimmune encephalitis, autoimmune-mediated hematologic disease), and the like.

### DETAILED DESCRIPTION

The technical solutions of the present disclosure will be further described in detail with reference to the following specific examples. It should be understood that the following examples are merely exemplary illustration and explanation of the present disclosure and should not be construed as limiting the protection scope of the present disclosure. All techniques implemented based on the content described above of the present disclosure are encompassed within the protection scope of the present disclosure.

Unless otherwise stated, the starting materials and reagents used in the following examples are all commercially available products or can be prepared by using known methods.

The experimental methods described in the following examples are conventional methods unless otherwise specified. The resulting compounds are determined for ¹H NMR spectra and mass spectra by using a Varian Mercury-Plus 400 nuclear magnetic resonance spectrometer and a Waters Q-TOF-Ultima mass spectrometer. The starting materials, reagents, and biomaterials are commercially available, unless otherwise specified.

Explanation of the abbreviations used in the following examples and elsewhere herein is as follows:

| Abbreviation | In English |
|---|---|
| BME | 2-Mercaptoethanonl |
| BnCl | Benzyl chloride |
| Boc | t-Butyloxy carbonyl |
| BOP | ((1*H*-Benzo[*d*][1,2,3]triazol-1-yl)oxy)tris(dimethylamino)phosphonium Hexafluorophosphate(V) |
| br | broad |
| CDCl₃ | Deuterated chlorofrom |
| CHCl₃ | Chlorofrom |
| d | doublet |
| DIEA | *N,N-*Diisopropylethylamine |
| DMAP | 4-Dimethylaminopyridine |
| DME | L,2-Dimethoxyethane |
| DMF | *N*,*N*-Dimethylformamide |
| DMSO | Dimethyl sulfoxide |
| EA | Ethyl acetate |
| EDCI | *N*-(3-Dimethylaminopropyl)-*N*'-ethylcarbodiimide hydrochloride |
| ESI | Electrospray Ionization |
| Et₃SiH | Triethylsilane |
| FCC | Flash Column Chromatography |
| h | hour |
| ¹H NMR | Proton Nuclear Magnetic Resonance |
| HATU | [bis(dimethylamino)methylene]-1*H*-1,2,3-triazolo[4,5-*b*]pyridinium 3-oxide hexafluorophosphate |
| HCO₂NH₄ | Ammonium formate |
| HOBt | Benzotriazol-1-ol |
| HPLC | High Performance Liquid Chromatography |
| IC₅₀ | Concentration of inhibitory 50% (enzyme) |
| *J* | Coupling constant |
| K₂CO₃ | Potassium carbonate |
| KI | Potassium iodide |
| m | multiplet |
| MeOH | Methanol |
| MHz | Megahertz |
| min | minute |
| NaH | Sodium hydride |
| NaCl | Sodium chloride |
| Na₂SO₄ | Sodium sulphate (anhyfrous) |
| Pd/C | Palladium on Carbon |
| PdCl₂ | Palladium chloride |
| Pd(OH)₂ | Palladium hydroxide |
| Pd(OAc)₂ | Palladium(II) acetate |
| PE | Petroleum Ether |
| Prionex | |
| q | quartet |
| r.t. | room temperature |
| s | singlet |
| t | triplet |
| TEA | Triethylamine |
| TFA | Trifluoroacetic Acid |
| Tfac | Trifluoroacetyl |
| Triton X-100 | |
| Ub-Rh110 | Ubiquitin-Rhodamine 110 |
| X-phos | 2-Dicyclohexylphosphino-2',4',6'-triisopropylbiphenyl |

### Synthesis Method for Compounds

The compounds of the present disclosure can be prepared by methods known in the art of organic synthesis, can be prepared from commercially available starting materials, or synthesized by using known organic, inorganic, and/or enzymatic methods. For example, the compounds of the present disclosure can be prepared using the general schemes described below. Carboxylic acid **I-A** and amine **I-B** are reacted with a peptide coupling reagent (such as EDCI-HOBt, BOP, or HATU) under basic conditions (such as DIEA, TEA, or DMAP) to form amide **I'**, then a halogen atom is replaced with hydrocarbylamino or hydrocarbyloxy, and a protective group (such as Tfac and/or Boc) is removed to give a target compound I: 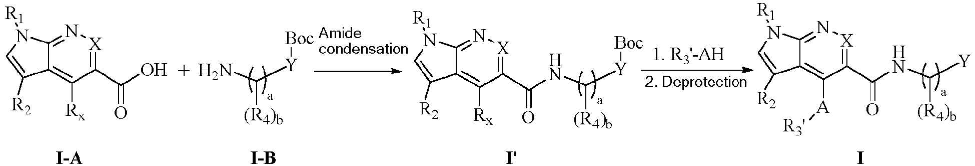 (Rₓ = Cl, Br; A = NH, O; R₁, R₂, R₄, a, b, X, and Y are as defined for those in formula **I**; R₃' is as defined for those in formulas **II-a** and **II-b**)

### Example 1

Preparation of compounds **I-01** (N-4-(3,8-diazabicyclo[3.2.1]octan-3-yl)phenethyl-4-chloro-1-ethyl-1H-pyrrolo[2,3-b]pyridine-5-carboxamide) and **1-02** (*N*-4-(3,8-diazabicyclo[3.2.1]octan-3-yl)phenethyl-1-ethyl-4-methylamino-1*H*-pyrrolo[2,3-b]pyridine-5-carboxamide):

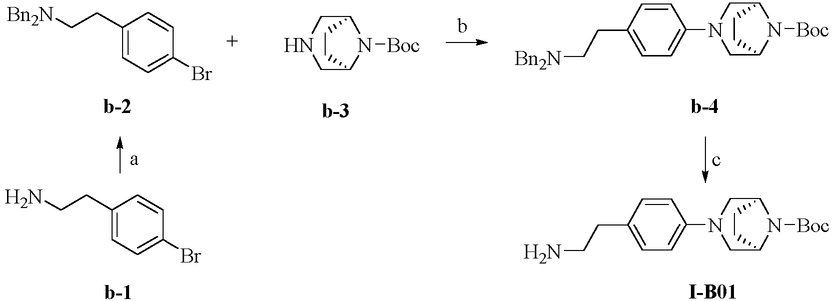

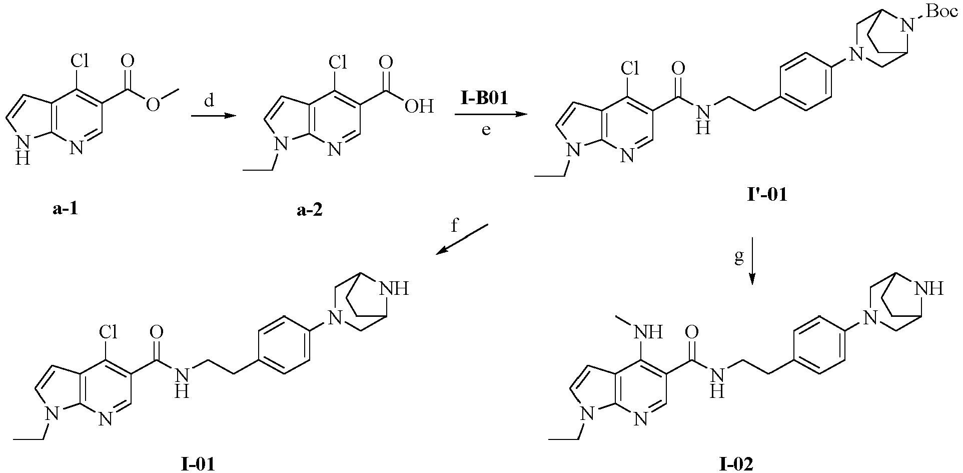

### Steps:

a) To a reaction flask (250 mL) were added *p*-bromophenylethylamine **b-1** (10.00 g, 50 mmol), KI (0.41 g, 2.5 mmol), K₂CO₃ (16.58 g, 120 mmol), and acetonitrile (100 mL), and the mixture was heated to reflux. BnCl (20.89 g, 165 mmol) was added dropwise. After the dropwise addition, the mixture was refluxed for 2 h. The reaction liquid was filtered to remove inorganic salts, and the filtrate was concentrated under reduced pressure to remove acetonitrile. Chloroform (200 mL) was added to the concentrate, and the mixture was washed with a saturated NaCl solution (100 mL × 2), dried over anhydrous Na₂SO₄, and concentrated under reduced pressure to give a crude product. The crude product was concentrated under reduced pressure to remove excess BnCl and by-product benzyl alcohol to give **b-2** (pale yellow liquid, 18.39 g, 97%).
b) To a reaction flask (250 mL) were added **b-3** (4.25 g, 20 mmol), **b-2** (9.13 g, 24 mmol), Pd(OAc)₂ (449 mg, 2 mmol), X-phos (953 mg, 2 mmol), Cs₂CO₃ (13.03 g, 40 mmol), and toluene (80 mL), and the mixture was purged with N₂ under vacuum, heated to 100 °C, and reacted for 18 h. The reaction liquid was filtered to remove insoluble substances, and the filtrate was concentrated under reduced pressure. The concentrate was subjected to silica gel column chromatography (gradient elution with PEÆA = 19:1-9:1) to give **b-4** (8.59 g, 84%).
c) To a reaction flask (250 mL) were added **b-4** (8.15 g, 15.9 mmol), HCO₂NH₄ (20.09 g, 318.5 mmol), Pd(OH)₂/C (2.26 g, 15% Pd), and MeOH (65 mL), and the mixture was purged with N₂ under vacuum, heated to 60 °C, and reacted overnight. The reaction liquid was filtered to remove insoluble substances, and the filtrate was concentrated under reduced pressure to remove methanol. Chloroform (200 mL) was added to the concentrate, and the mixture was washed with a saturated NaCl solution (50 mL × 3), dried over anhydrous Na₂SO₄, and concentrated under reduced pressure to give a crude product. The crude product was subjected to silica gel column chromatography (gradient elution with CHCl₃-MeOH 20:1→8:2) to give **I-B01** (5.15 g, 93%). ESI-MS: *m*/*z* 332 ([M+H]⁺).
d) To a reaction flask (50 mL) were added **a-1** (1053 mg, 5.0 mmol) and dry DMF (10 mL), and the mixture was cooled to 0 °C. 60% NaH (240 mg, 6.0 mmol) was added, and the mixture was reacted for 30 min. Then EtI (936 mg, 6.0 mmol) was added dropwise over 5 min at 0 °C. After the dropwise addition, the mixture was reacted at room temperature for 1 h. Water (10 mL) and a 10 *N* NaOH solution (1.0 mL) were added to the reaction liquid, and the mixture was heated to 60 °C and reacted for 1 h. The reaction liquid was cooled to room temperature, and then water (20 mL) was added. The reaction liquid was cooled to 0 °C and adjusted to about pH 3 by adding 18% hydrochloric acid dropwise, and a white solid was precipitated. The mixture was successively stirred at room temperature for 15 min, filtered under vacuum, and washed with water until a neutral filtrate was obtained. The crude product was recrystallized from 35 mL of ethanol to give **a-2** (white solid, 934 mg, 83.2%).
e) To a reaction flask (10 mL) were added **a-2** (225 mg, 1.0 mmol), **I-B01** (331 mg, 1.0 mmol), HOBt (149 mg, 1.1 mmol), and EDCI (230 mg, 1.2 mmol), followed by anhydrous DMF (4 mL) and DIEA (524 *µ*L, 3.0 mmol), and the mixture was reacted at room temperature overnight. The reaction liquid was concentrated under reduced pressure to give a crude product, which was subjected to silica gel column chromatography (25 g, elution with PE/EtOAc = 1:1) to give **I'-01** (off-white solid, 439 mg, 81.6%).
f) **I'-01** (54 mg, 0.1 mmol) and TFA (150 *µ*L, 10 mmol) were stirred in anhydrous CHCl₃ (2 mL) for 30 min. The reaction liquid was concentrated under reduced pressure to remove the solvent, and the concentrate was separated by silica gel column chromatography (20 g, CHCl₃/MeOH/NH₃·H₂O = 9:1:0.05) to give **I-01** (off-white solid, 38 mg, 86.5%). ¹H NMR (CDCl₃, 400 MHz) *δ:* 8.58 (s, 1H), 7.29 (d, *J* = 3.5 Hz, 1H), 7.16 (d, *J* = 8.6 Hz, 2H), 6.79 (d, *J* = 8.6 Hz, 2H), 6.58 (d, *J* = 3.5 Hz, 1H), 6.35 (t, *J =* 5.5 Hz, 1H), 4.32 (q, *J =* 7.3 Hz, 2H), 4.14 (br s, 2H), 3.73 (q, *J =* 6.4 Hz, 2H), 3.47 (br d, *J* = 12.0 Hz, 2H), 3.42 (br d, *J =* 12.0 Hz, 2H), 2.90 (t, *J =* 6.8 Hz, 2H), 2.24 (m, 2H), 2.05 (m, 2H), 1.46 (t, *J =* 7.3 Hz, 3H). ESI-MS *m*/*z*: 438 ([M+H]⁺).
g) To a thick-wall pressure-resistant reaction flask (15 mL) were added **I'-01** (108 mg, 0.2 mmol), a 40% aqueous methylamine solution (173 *µ*L, 2 mmol), and 1,4-dioxane (2 mL), and the mixture was heated to 100 °C and reacted for 6-10 h. The reaction liquid was concentrated under reduced pressure to remove the solvent. Anhydrous CHCl₃ (1 mL) and TFA (297 *µ*L, 20 mmol) were added, and the mixture was heated to 60 °C and reacted for 2 h. The reaction liquid was concentrated under reduced pressure to remove the solvent, and subjected to silica gel column chromatography (20 g, elution with CHCl₃/MeOH/NH₃·H₂O = 9:1: 0.05) to give **I-02** (pale yellow gum, 59 mg, 67.0%). ¹H NMR (DMSO-*d*₆, 400 MHz) *δ*: 8.97 (q, *J =*5.3 Hz, 1H), 8.28 (t, *J =* 5.4 Hz, 1H), 8.25 (s, 1H), 7.22 (d, *J =* 3.6 Hz, 1H), 7.09 (d, *J =* 8.6 Hz, 2H), 6.81 (d, *J =* 8.6 Hz, 2H), 6.75 (d, *J =* 3.6 Hz, 1H), 4.17 (q, *J =* 7.2 Hz, 2H), 3.96 (br s, 2H), 3.51 (br d, *J =* 11.1 Hz, 2H), 3.37 (q, *J =* 6.8 Hz, 2H), 3.22 (d, *J* = 5.4 Hz, 3H), 2.97 (br d, *J =* 11.1 Hz, 2H), 2.73 (t, *J =* 7.4 Hz, 2H), 1.94 - 1.84 (m, 4H), 1.32 (t, *J* = 7.2 Hz, 3H). ESI-MS *m*/*z* 433 ([M+H]⁺).

Example compounds **I-03** to **I-33** in Table 1 were synthesized by using appropriate synthesis precursors, with reference to the reagents and reaction conditions described above for Example 1 (compounds **I-01** and **I-02**).

**Table 1. Structures of Examples I-03 to I-33, and spectral characterization signals thereof**

| No. | Structure | ¹H NMR shift (multiplicity, coupling constant, integration). ESI-MS [M+H]⁺ *m*/*z* |
|---|---|---|
| **I-03** | 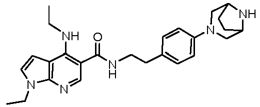 | (DMSO-*d*₆, 400 MHz) *δ:* 8.99 (q, *J =*5.5 Hz, 1H), 8.29 (t, *J =* 5.4 Hz, 1H), 8.25 (s, 1H), 7.22 (d, *J =* 3.6 Hz, 1H), 7.09 (d, *J =* 8.6 Hz, 2H), 6.81 (d, *J =* 8.6 Hz, 2H), 6.79 (d, *J* = 3.6 Hz, 1H), 4.17 (q, *J* = 7.2 Hz, 2H), 3.98 (br s, 2H), 3.51 (br d, *J* = 11.2 Hz, 2H), 3.38 (q, *J* = 6.9 Hz, 2H), 2.97 (br d, *J* = 11.0 Hz, 2H), 2.90 (quint, *J =* 6.8 Hz, 2H), 2.73 (t, *J =* 7.4 Hz, 2H), 1.94 - 1.84 (m, 4H), 1.35 (t, *J* =7.1 Hz, 3H), 1.32 (t, *J* = 7.2 Hz, 3H). ESI-MS *m*/*z* 447 ([M+H]⁺). |
| **I-04** | 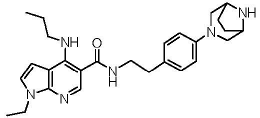 | (DMSO-*d*₆, 400 MHz) *δ:* 8.98 (q, *J =*5.3 Hz, 1H), 8.28 (t, *J =* 5.4 Hz, 1H), 8.25 (s, 1H), 7.22 (d, *J =* 3.6 Hz, 1H), 7.09 (d, *J =* 8.6 Hz, 2H), 6.81 (d, *J =* 8.6 Hz, 2H), 6.80 (d, *J* = 3.6 Hz, 1H), 4.17 (q, *J* = 7.2 Hz, 2H), 3.96 (br s, 2H), 3.50 (br d, *J =* 11.3 Hz, 2H), 3.37 (q, *J =* 6.8 Hz, 2H), 2.95 (br d, *J =* 11.3 Hz, 2H), 2.93 (q, *J =* 6.8 Hz, 2H), 2.73 (t, *J* = 7.4 Hz, 2H), 1.94 - 1.84 (m, 6H), 1.69 (m, 2H), 1.32 (t, *J =* 7.2 Hz, 3H), 0.94 (t, *J =* 7.2 Hz, 3H). ESI-MS *m*/*z* 461 ([M+H]⁺). |
| **I-05** | 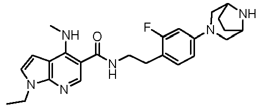 | (DMSO-*d*₆, 400 MHz) *δ:* 9.66 (m, 2H), 8.97 (q, *J* =5.3 Hz, 1H), 8.28 (t, *J* = 5.4 Hz, 1H), 8.25 (s, 1H), 7.22 (dd, *J* = 10.0, 9.0 Hz, 1H), 7.22 (d, *J =* 3.6 Hz, 1H), 6.75 (d, *J =* 3.6 Hz, 1H), 6.70 (dd, *J =* 16.4, 2.0 Hz, 1H), 6.67 (dd, *J =* 9.0, 2.0 Hz, 1H), 4.17 (q, *J* = 7.2 Hz, 2H), 4.09 (br s 2H), 3.61 (br d, *J* = 11.4 Hz, 2H), 3.42 (q, *J =* 6.8 Hz, 2H), 3.22 (d, *J =* 5.3 Hz, 3H), 3.15 (br d, *J =* 11.5 Hz, 2H), 2.88 (t, *J* = 7.4 Hz, 2H), 2.00 (m, 2H), 1.90 (m, 2H), 1.32 (t, *J =* 7.2 Hz, 3H). ESI-MS *m*/*z* 451 ([M+H]⁺). |
| **I-06** | 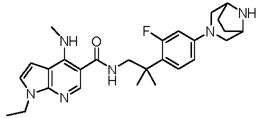 | (DMSO-*d*₆, 400 MHz) *δ*: 9.96 (br s, 1H), 9.66 (m, 2H), 9.10 (br s), 8.42 (s, 1H), 7.45 (d, *J =* 3.6 Hz, 1H), 7.16 (dd, *J =* 9.8, 8.8 Hz, 1H), 7.04 (d, *J =* 3.6 Hz, 1H), 6.70 (dd, *J* = 16.2, 2.2 Hz, 1H), 6.64 (dd, *J* = 8.8, 2.2 Hz, 1H), 4.41 (q, *J =* 7.2 Hz, 2H), 4.10 (br s 2H), 3.62 (br d, *J* = 11.4 Hz, 2H), 3.51 (br d, *J =* 6.2 Hz, 2H), 3.33 (br s, 3H), 3.15 (br d, *J =* 11.5 Hz, 2H), 2.00 (m, 2H), 1.90 (m, 2H), 1.35 (t, *J =* 7.2 Hz, 3H), 1.29 (s, 6H). ESI-MS *m*/*z* 479 ([M+H]⁺). |
| **I-07** | 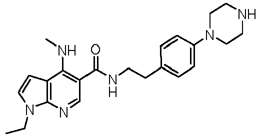 | (DMSO-*d*₆, 400 MHz) *δ:* 8.96 (q, *J =*5.5 Hz, 1H), 8.28 (t, *J =* 5.3 Hz, 1H), 8.25 (s, 1H), 7.21 (d, *J =* 3.6 Hz, 1H), 7.09 (d, *J =* 8.4 Hz, 2H), 6.85 (d, *J =* 8.4 Hz, 2H), 6.74 (d, *J =* 3.6 Hz, 1H), 4.18 (q, *J =* 7.2 Hz, 2H), 3.50-3.41 (m, 2H), 3.22 (d, *J =* 5.5 Hz, 3H), 3.00-2.96 (m, 4H), 2.84-2.72 (m, 6H), 1.32 (t, *J =* 7.2 Hz, 3H). ESI-MS [M+H]⁺ *m*/*z* 407. |
| **I-08** | 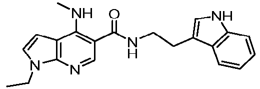 | (CDCl₃, 400 MHz) *δ*: 8.48 (s, 1H), 8.10 (q, *J =* 5.5 Hz, 1H), 7.66 (br d, *J =* 7.9 Hz, 1H), 7.38 (br d, *J* = 8.1 Hz, 1H), 7.30 (d, *J* = 3.6 Hz, 1H), 7.22 (br t, *J =* 7.3 Hz, 1H), 7.14 (br t, *J=* 7.2 Hz, 1H), 7.09 (d, *J =* 2.0 Hz, 1H), 6.67 (d, *J =* 3.6 Hz, 1H), 6.41 (t, *J =* 5.5 Hz, 1H), 4.33 (q, *J =* 7.3 Hz, 2H), 3.73 (q, *J =* 6.2 Hz, 2H), 3.32 (d, *J =* 5.5 Hz, 3H), 3.08 (t, *J =* 6.7 Hz, 2H), 1.46 (t, *J =* 7.3 Hz, 3H). ESI-MS *m*/*z* 362 ([M+H]⁺). |
| **I-09** | 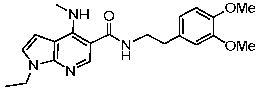 | (CDCl₃, 400 MHz) *δ:* 8.41 (s, 1H), 8.21 (q, *J =* 5.5 Hz, 1H), 7.29 (d,*J* = 3.6 Hz, 1H), 6.79 (d, *J =* 8.0 Hz, 1H), 6.74 (d, *J =* 1.8 Hz, 1H), 6.66 (d, *J* = 3.6 Hz, 1H), 6.60 (dd, *J =* 8.0, 1.8 Hz, 1H), 6.32 (t, *J =* 5.2 Hz, 1H), 4.32 (q, *J* = 7.3 Hz, 2H), 3.83 (s, 3H), 3.75 (s, 3H), 3.39 (td, *J =* 6.7, 5.0 Hz, 2H), 3.32 (d, *J =* 5.5 Hz, 3H), 2.78 (t, *J =* 6.7 Hz, 2H), 1.46 (t, *J =* 7.3 Hz, 3H). ESI-MS *m*/*z* 383 ([M+H]⁺). |
| **I-10** | 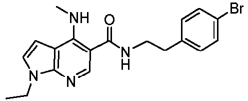 | (CDCl₃, 400 MHz) *δ*: 8.45 (s, 1H), 8.12 (q, *J* = 5.7 Hz, 1H), 7.82 (d, *J* = 8.3 Hz, 2H), 7.30 (d, *J =* 3.6 Hz, 1H), 7.09 (d, *J =* 8.3 Hz, 2H), 6.66 (d, *J =* 3.6 Hz, 1H), 6.35 (t, *J =* 5.5 Hz, 1H), 4.32 (q, *J* = 7.3 Hz, 2H), 3.41 (q, *J* = 6.7, 5.0 Hz, 2H), 3.32 (d, *J* = 5.7 Hz, 3H), 2.80 (t, *J* = 6.7 Hz, 2H), 1.46 (t, *J* = 7.3 Hz, 3H).ESI-MS *m*/*z* 401 ([M+H]⁺). |
| **I-11** | 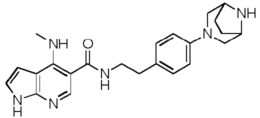 | (DMSO-*d*₆, 400 MHz) *δ*: 11.41 (s, 1H), 9.00 (q, *J =* 5.3 Hz, 1H), 8.24 (s, 1H), 8.24 (t, *J =* 5.5 Hz, 1H), 7.09 (d, *J =* 3.5 Hz, 1H), 7.05 (d, *J =* 8.6 Hz, 2H), 6.73 (d, *J =* 3.5 Hz, 1H), 6.73 (d, *J =* 8.6 Hz, 2H), 3.56 (br s, 2H), 3.40 - 3.33 (m, 4H), 3.23 (d, *J* = 5.3 Hz, 3H), 2.75 - 2.68 (m, 4H), 1.75 - 1.68 (m, 4H). ESI-MS *m*/*z* 405 ([M+H]⁺). |
| **I-12** | 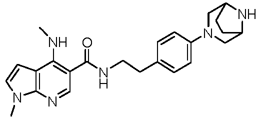 | (DMSO-*d*₆, 400 MHz) *δ*: 9.00 (q, *J =* 5.3 Hz, 1H), 8.27 (s, 1H), 8.27 (t, *J* = 5.5 Hz, 1H), 7.15 (d, *J =* 3.5 Hz, 1H), 7.04 (d, *J* = 8.6 Hz, 2H), 6.74 (d, *J* = 3.5 Hz, 1H), 6.72 (d, *J* = 8.6 Hz, 2H), 3.70 (s, 3H), 3.50 (br s, 2H), 3.40 - 3.32 (m, 4H), 3.23 (d, *J =* 5.3 Hz, 3H), 2.74 - 2.66 (m, 4H), 1.73 - 1.62 (m, 4H). ESI-MS *m*/*z* 419 ([M+H]⁺). |
| **I-13** | 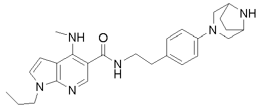 | (DMSO-*d*₆, 400 MHz) *δ:* δ 8.96 (q, *J =* 5.3 Hz, 1H), 8.25 (t, *J =* 5.5 Hz, 1H), 8.24 (s, 1H), 7.19 (d, *J* = 3.6 Hz, 1H), 7.04 (d, *J* = 8.6 Hz, 2H), 6.75 (d, *J =* 3.6 Hz, 1H), 6.72 (d, *J* = 8.6 Hz, 2H), 4.10 (t, *J* = 7.0 Hz, 2H), 3.50 (br s, 2H), 3.40 - 3.30 (m, 4H), 3.23 (d, *J =* 5.3 Hz, 3H), 2.75 - 2.65 (m, 4H), 1.75 (sext, *J =* 7.2 Hz, 2H), 1.71 - 1.62 (m, 4H), 0.82 (t, *J* = 7.4 Hz, 3H). ESI-MS *m*/*z* 447 ([M+H]⁺). |
| **I-14** | 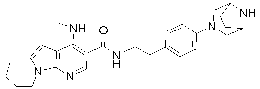 | (DMSO-*d*₆, 400 MHz) *δ:* 8.96 (q, *J =* 5.3 Hz, 1H), 8.25 (t, *J =* 5.5 Hz, 1H), 8.24 (s, 1H), 7.19 (d, *J =* 3.6 Hz, 1H), 7.04 (d, *J =* 8.5 Hz, 2H), 6.74 (d, *J* = 3.6 Hz, 1H), 6.72 (d, *J* = 8.5 Hz, 2H), 4.14 (t, *J* = 7.0 Hz, 2H), 3.49 (br s, 2H), 3.40 - 3.31 (m, 4H), 3.23 (d, *J =* 5.3 Hz, 4H), 1.72 (quint, *J =* 7.4 Hz, 2H), 1.69 - 1.61 (m, 4H), 1.23 (sext, *J* =7.4 Hz, 2H), 0.88 (t, *J =* 7.4 Hz, 3H). ESI-MS *m*/*z* 461 ([M+H]⁺). |
| **I-15** | 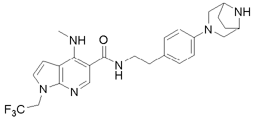 | (DMSO-*d*₆, 400 MHz) *δ*: 9.35 (m, 1H), 8.77 (q, *J* =5.4 Hz, 1H), 8.34 (t, *J* = 5.3 Hz, 1H), 8.28 (s, 1H), 7.28 (d, *J* = 3.6 Hz, 1H), 7.10 (d, *J* = 8.4 Hz, 2H), 6.86 (d, *J* = 8.4 Hz, 2H), 6.79 (d, *J* = 3.6 Hz, 1H), 5.10-5.03 (m, 2H), 3.98 (br s, 2H), 3.52 (br d, *J =* 11.6 Hz, 2H), 3.37 (q, *J =* 6.8 Hz, 2H), 3.22 (d, *J =* 5.4 Hz, 3H), 2.97 (br d, *J =* 11.5 Hz, 2H), 2.73 (t, *J =* 7.4 Hz, 2H), 1.94 - 1.84 (m, 4H). ESI-MS *m*/*z* 487 ([M+H]⁺). |
| **I-16** | 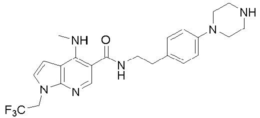 | (DMSO-*d*₆, 400 MHz) *δ*: 8.98 (q, *J* =5.4 Hz, 1H), 8.29 (t, *J =* 5.3 Hz, 1H), 8.29 (s, 1H), 7.28 (d, *J =* 3.6 Hz, 1H), 7.09 (d, *J =* 8.4 Hz, 2H), 6.85 (d, *J =* 8.4 Hz, 2H), 6.78 (d, *J* = 3.6 Hz, 1H), 5.10-5.03 (m, 2H), 3.50-3.41 (m, 2H), 3.22 (d, *J* = 5.4 Hz, 3H), 3.00-2.96 (m, 4H), 2.84-2.72 (m, 6H). ESI-MS *m*/*z* 461 ([M+H]⁺). |
| **I-17** | 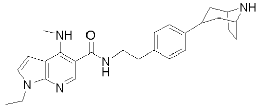 | (Methanol-*d*₄, 400 MHz) *δ:* 8.16 (s, 1H), 7.23-7.18 (m, 4H), 7.10 (d, *J =* 3.6 Hz, 1H), 6.62 (d, *J* = 3.6 Hz, 1H), 3.98 (q, *J* = 7.2 Hz, 2H), 3.68 (br s, 2H), 3.50 (t, *J* = 7.5 Hz, 2H), 3.41 (m, 2H), 3.27 (s, 3H), 2.99 (m, 1H), 2.83 (t, *J* = 7.5 Hz, 2H), 2.38-2.25 (m, 2H), 1.95-1.88 (m, 2H), 1.76-1.72 (m, 2H), 1.70-1.64 (m, 2H), 1.26 (t, *J* = 7.2 Hz, 3H). ESI-MS *m*/*z* 432 ([M+H]⁺). |
| **I-18** | 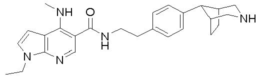 | (CDCl₃, 400 MHz) *δ:* 8.46 (s, 1H), 8.09 (q, *J* = 5.7 Hz, 1H), 7.29 (d, *J* = 3.5 Hz, 1H), 7.21 (m, 4H), 6.66 (d, *J* = 3.5 Hz, 1H), 6.35 (t, *J* = 5.5 Hz, 1H), 4.32 (q, *J = 7.3* Hz, 2H), 3.61 (q, *J =* 6.6 Hz, 2H), 3.41 (dd, *J =* 11.5, 3.4 Hz, 2H), 3.32 (d, *J =* 5.7 Hz, 3H), 3.10 (dd, *J =* 11.5, 2.7 Hz, 2H), 3.01 (br t, *J =* 3.0 Hz, 1H), 2.85 (t, *J* = 7.0 Hz, 2H), 1.74-1.65 (m, 6H), 1.46 (t, *J* = 7.3 Hz, 3H). ESI-MS *m*/*z* 432 ([M+H]⁺). |
| **I-19** | 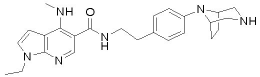 | (CDCl₃, 400 MHz) *δ:* 8.45 (s, 1H), 8.10 (q, *J* = 5.7 Hz, 1H), 7.30 (d, *J* = 3.6 Hz, 1H), 7.15 (d, *J* = 8.6 Hz, 2H), 6.80 (d, *J* = 8.6 Hz, 2H), 6.67 (d, *J =* 3.6 Hz, 1H), 6.35 (t, *J =* 5.5 Hz, 1H), 4.32 (q, *J =* 7.3 Hz, 2H), 4.01 (m, 2H), 3.61 (q, *J =* 6.5 Hz, 2H), 3.32 (d, *J* = 5.7 Hz, 3H), 3.06 (br d, *J* = 11.5, 2.9 Hz, 2H), 2.97 (dd, *J* = 11.5, 2.0 Hz, 2H), 2.82 (t, *J =* 6.9 Hz, 2H), 1.88-1.80 (m, 4H), 1.46 (t, *J =* 7.3 Hz, 3H). ESI-MS *m*/*z* 433 ([M+H]⁺). |
| **I-20** | 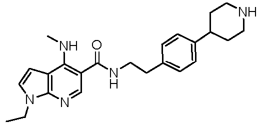 | (DMSO-*d*₆, 400 MHz) *δ:* 8.95 (q, *J =*5.5 Hz, 1H), 8.26 (t, *J* = 5.4 Hz, 1H), 8.25 (s, 1H), 7.20 (d, *J =* 3.6 Hz, 1H), 7.18 (m, 4H), 6.73 (d, *J =* 3.6 Hz, 1H), 4.17 (q, *J* = 7.2 Hz, 2H), 3.39 (td, *J* = 6.9, 5.6 Hz, 2H), 3.26 (m, 2H), 3.22 (d, *J =* 5.5 Hz, 3H), 2.88-2.81 (m, 4H), 2.73 (m, 1H), 1.89 (m, 2H), 1.69 (m, 2H), 1.32 (t, *J =* 7.2 Hz, 3H). ESI-MS *m*/*z* 406 ([M+H]⁺). |
| **I-21** | 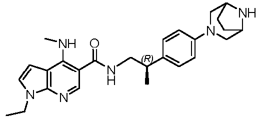 | (CDCl₃) *δ* 8.45 (s, 1H), 8.10 (q, *J =* 5.7 Hz, 1H), 7.30 (d, *J =* 3.6 Hz, 1H), 7.25 (d, *J =* 8.6 Hz, 2H), 6.86 (d, *J =* 8.6 Hz, 2H), 6.67 (d, *J =* 3.6 Hz, 1H), 6.35 (t, *J =* 5.5 Hz, 1H), 4.32 (q, *J =* 7.3 Hz, 2H), 3.78 (br s, 2H), 3.66 (m, 2H), 3.47 (dd, *J =* 11.4, 2.3 Hz, 2H), 3.32 (d, *J =* 5.7 Hz, 3H), 3.02 (dd, *J =* 11.3, 1.5 Hz 2H), 2.92 (m), 1.94 (br s, 4H),1.46 (t, *J* = 7.3 Hz, 3H), 1.25 (d, *J* = 6.8 Hz, 3H). ESI-MS *m*/*z* 447 ([M+H]⁺). |
| **I-22** | 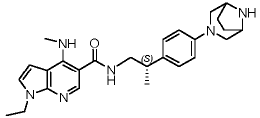 | (CDCl₃) *δ* 8.45 (s, 1H), 8.10 (q, *J =* 5.7 Hz, 1H), 7.30 (d, *J =* 3.6 Hz, 1H), 7.25 (d, *J =* 8.6 Hz, 2H), 6.86 (d, *J =* 8.6 Hz, 2H), 6.67 (d, *J =* 3.6 Hz, 1H), 6.35 (t, *J =* 5.5 Hz, 1H), 4.32 (q, *J =* 7.3 Hz, 2H), 3.78 (br s, 2H), 3.66 (m, 2H), 3.47 (dd, *J =* 11.4, 2.3 Hz, 2H), 3.32 (d, *J =* 5.7 Hz, 3H), 3.02 (dd, *J =* 11.3, 1.5 Hz 2H), 2.92 (m), 1.94 (br s, 4H),1.46 (t, *J* = 7.3 Hz, 3H), 1.25 (d, *J =* 6.8 Hz, 3H). ESI-MS *m*/*z* 447 ([M+H]⁺). |
| **I-23** | 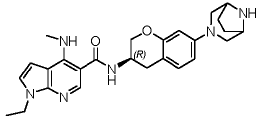 | (DMSO-*d*₆, 400 MHz) *δ:* 8.93 (q, *J =* 5.5 Hz, 1H), 8.28 (s, 1H), 7.75 (d, *J* = 7.4 Hz, 1H), 7.20 (d, *J =* 3.6 Hz, 1H), 6.95 (d, *J =* 8.5 Hz, 1H), 6.73 (d, *J* = 3.6 Hz, 1H), 6.47 (dd, *J =* 8.5, 2.4 Hz, 1H), 6.31 (d, *J =* 2.4 Hz, 1H), 4.28 (m, 1H), 4.19 (q, *J =* 7.2 Hz, 2H), 4.15 (dd, *J =* 10.3, 3.4 Hz, 1H), 4.08 (br s, 2H), 3.83 (t, *J* = 9.8 Hz, 1H), 3.54 (dd, *J =* 10.3, 6.1 Hz, 2H), 3.22 (d, *J* = 5.5 Hz, 3H), 3.05 (br d, *J =* 11.7 Hz, 2H), 2.86 (d, *J* = 7.3 Hz, 2H), 2.00 - 1.93 (m, 2H), 1.91 (dd, *J =* 10.9, 6.5 Hz, 2H), 1.32 (t, *J =* 7.2 Hz, 3H). ESI-MS *m*/*z* 464 ([M+H]⁺). |
| **I-24** | 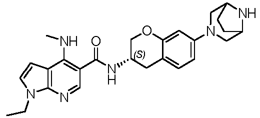 | (DMSO-*d*₆, 400 MHz) *δ*: 8.93 (q, *J =* 5.5 Hz, 1H), 8.28 (s, 1H), 7.75 (d, *J* = 7.4 Hz, 1H), 7.20 (d, *J =* 3.6 Hz, 1H), 6.95 (d, *J =* 8.5 Hz, 1H), 6.73 (d, *J* = 3.6 Hz, 1H), 6.47 (dd, *J =* 8.5, 2.4 Hz, 1H), 6.31 (d, *J =* 2.4 Hz, 1H), 4.28 (m, 1H), 4.19 (q, *J =* 7.2 Hz, 2H), 4.15 (dd, *J =* 10.3, 3.4 Hz, 1H), 4.08 (br s, 2H), 3.83 (t, *J* = 9.8 Hz, 1H), 3.54 (dd, *J =* 10.3, 6.1 Hz, 2H), 3.22 (d, *J* = 5.5 Hz, 3H), 3.05 (br d, *J =* 11.7 Hz, 2H), 2.86 (d, *J* = 7.3 Hz, 2H), 2.00 - 1.93 (m, 2H), 1.91 (dd, *J =* 10.9, 6.5 Hz, 2H), 1.32 (t, *J =* 7.2 Hz, 3H). ESI-MS *m*/*z* 464 ([M+H]⁺). |
| **I-25** | 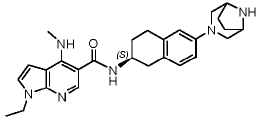 | (DMSO-*d*₆, 400 MHz) *δ*: 9.05 (q, *J* = 5.5 Hz, 1H), 8.31 (s, 1H), 7.88 (d, *J* = 7.4 Hz, 1H), 7.20 (d, *J =* 3.6 Hz, 1H), 6.94 (d, *J =* 8.5 Hz, 1H), 6.73 (d, *J* = 3.6 Hz, 1H), 6.69 (dd, *J* = 8.5, 2.4 Hz, 1H), 6.61 (d, *J =* 2.4 Hz, 1H), 4.19 (q, *J* = 7.2 Hz, 2H), 4.16 (m, 1H), 4.09 (br s, 2H), 3.53 (dd, *J =* 12.2, 5.1 Hz, 2H), 3.22 (d, *J* = 5.5 Hz, 3H), 3.08 (d, *J* = 12.2 Hz, 2H), 2.86 (dd, *J* = 15.8, 5.1 Hz, 1H), 2.80 (dd, *J =* 8.3, 4.3 Hz, 2H), 2.73 (dd, *J =* 15.8, 10.8 Hz, 1H), 2.60 (s, 3H), 2.00 - 1.94 (m, 3H), 1.91 (m, 2H), 1.75 (tt, 11.8, 8.9 Hz, 1H),1.33 (t, *J = 7.2* Hz, 3H). ESI-MS *m*/*z* 462 ([M+H]⁺). |
| **I-26** | 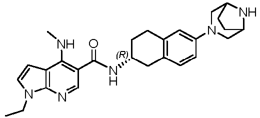 | (DMSO-*d*₆, 400 MHz) *δ:* 9.05 (q, *J* = 5.5 Hz, 1H), 8.31 (s, 1H), 7.88 (d, *J* = 7.4 Hz, 1H), 7.20 (d, *J =* 3.6 Hz, 1H), 6.94 (d, *J =* 8.5 Hz, 1H), 6.73 (d, *J* = 3.6 Hz, 1H), 6.69 (dd, *J* = 8.5, 2.4 Hz, 1H), 6.61 (d, *J =* 2.4 Hz, 1H), 4.19 (q, *J* = 7.2 Hz, 2H), 4.16 (m, 1H), 4.09 (br s, 2H), 3.53 (dd, *J =* 12.2, 5.1 Hz, 2H), 3.22 (d, *J* = 5.5 Hz, 3H), 3.08 (d, *J* = 12.2 Hz, 2H), 2.86 (dd, *J* = 15.8, 5.1 Hz, 1H), 2.80 (dd, *J =* 8.3, 4.3 Hz, 2H), 2.73 (dd, *J =* 15.8, 10.8 Hz, 1H), 2.60 (s, 3H), 2.00 - 1.94 (m, 3H), 1.91 (m, 2H), 1.75 (tt, 11.8, 8.9 Hz, 1H),1.33 (t, *J =* 7.2 Hz, 3H). ESI-MS *m*/*z* 462 ([M+H]⁺). |
| **I-27** | 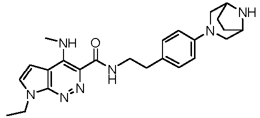 | (DMSO-*d*₆, 400 MHz) *δ:* 9.05 (q, *J* =5.4 Hz, 1H), 8.33 (t, *J =* 5.6 Hz, 1H), 7.44 (d, *J =* 3.5 Hz, 1H), 7.05 (d, *J =* 8.5 Hz, 2H), 6.78 (d, *J =* 8.5 Hz, 2H), 6.72 (d, *J =* 3.5 Hz, 1H), 4.26 (q, *J =* 7.2 Hz, 2H), 3.98 (br s, 2H), 3.50 (br d, *J* = 11.6 Hz, 2H), 3.37 (q, *J =* 6.9 Hz, 2H), 3.22 (d, *J =* 5.4 Hz, 3H), 2.97 (br d, *J =* 11.7 Hz, 2H), 2.75 (t, *J =* 7.2 Hz, 2H), 1.95 - 1.85 (m, 4H), 1.40 (t, *J =* 7.2 Hz, 3H). ESI-MS *m*/*z* 434 ([M+H]⁺). |
| **I-28** | 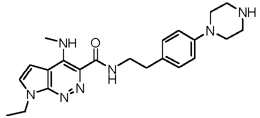 | (DMSO-*d*₆, 400 MHz) *δ:* 9.02 (q, *J =*5.5 Hz, 1H), 8.53 (t, *J =* 5.4 Hz, 1H), 7.46 (d, *J =* 3.6 Hz, 1H), 7.09 (d, *J =* 8.4 Hz, 2H), 6.85 (d, *J =* 8.4 Hz, 2H), 6.73 (d, *J =* 3.6 Hz, 1H), 4.27 (q, *J =* 7.2 Hz, 2H), 3.50-3.42 (m, 2H), 3.23 (d, *J =* 5.4 Hz, 3H), 3.00-2.96 (m, 4H), 2.84-2.72 (m, 6H), 1.39 (t, *J =* 7.2 Hz, 3H). ESI-MS *m*/*z* 408 ([M+H]⁺). |
| **I-29** | 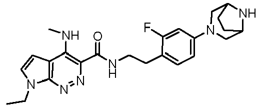 | (DMSO-*d*₆, 400 MHz) *δ:* 9.58 (m, 2H), 8.85 (q, *J =*5.3 Hz, 1H), 8.34 (t, *J* = 5.4 Hz, 1H), 7.47 (d, *J =* 3.6 Hz, 1H), 7.24 (dd, *J =* 10.0, 9.0 Hz, 1H), 7.22 (d, *J* = 3.6 Hz, 1H), 6.75 (d, *J =* 3.6 Hz, 1H), 6.70 (dd, *J =* 16.4, 2.0 Hz, 1H), 6.67 (dd, *J =* 9.0, 2.0 Hz, 1H), 4.20 (q, *J =* 7.2 Hz, 2H), 4.08 (br s 2H), 3.60 (br d, *J =* 11.5 Hz, 2H), 3.42 (q, *J =* 6.9 Hz, 2H), 3.23 (d, *J =* 5.3 Hz, 3H), 3.15 (br d, *J =* 11.5 Hz, 2H), 2.89 (t, *J =* 7.4 Hz, 2H), 2.00 (m, 2H), 1.90 (m, 2H), 1.33 (t, *J =* 7.2 Hz, 3H). ESI-MS [M+H]⁺ *m*/*z* 452. |
| I-30 | 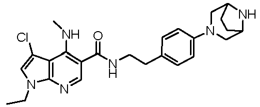 | (CDCl₃, 400 MHz) *δ:* 8.77 (q, *J* =5.4 Hz, 1H), 8.62 (s, 1H), 7.54 (s, 1H), 7.18 (d, *J* = 8.4 Hz, 2H), 6.80 (d, *J* = 8.4 Hz, 2H), 6.35 (t, *J* = 5.5 Hz, 1H), 4.33 (q, *J =* 7.2 Hz, 2H), 4.12 (br s, 2H), 3.75 (q, *J =* 6.4 Hz, 2H), 3.48 (br d, *J* = 12.0 Hz, 2H), 3.43 (br d, *J =* 12.0 Hz, 2H), 3.24 (d, *J =* 5.5 Hz, 3H), 2.88 (t, *J* = 6.8 Hz, 2H), 2.25 (m, 2H), 2.06 (m, 2H), 1.47 (t, *J* = 7.2 Hz, 3H). ESI-MS *m*/*z* 467 ([M+H]⁺). |
| **I-31** | 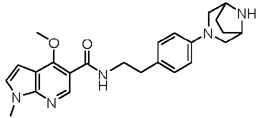 | (DMSO-*d*₆, 400 MHz) *δ:* 9.98 (br s, 1H), 8.53 (t, *J* = 5.2 Hz, 1H), 8.41 (s, 1H), 7.51 (d, *J =* 3.7 Hz, 1H), 7.09 (d, *J* = 8.5 Hz, 2H), 6.90 (d, *J =* 3.7 Hz, 1H), 6.80 (d, *J =* 8.5 Hz, 2H), 4.35 (s, 3H), 3.95 (br s, 2H), 3.69 (s, 3H), 3.51 (br d, *J =* 11.4 Hz, 2H), 3.37 (td, *J =* 7.4, 5.4 Hz, 2H), 2.97 (br d, *J =* 11.5 Hz, 2H), 2.73 (t, *J =* 7.4 Hz, 2H), 1.94 - 1.84 (m, 4H). ESI-MS *m*/*z* 420 ([M+H]⁺). |
| **I-32** | 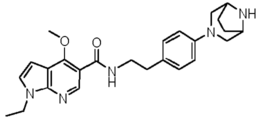 | (DMSO-*d*₆, 400 MHz) *δ:* 9.95 (br s, 1H), 8.56 (t, *J* = 5.2 Hz, 1H), 8.43 (s, 1H), 7.50 (d, *J =* 3.6 Hz, 1H), 7.09 (d, *J =* 8.6 Hz, 2H), 6.90 (d, *J =* 3.6 Hz, 1H), 6.81 (d, *J* = 8.6 Hz, 2H), 4.35 (s, 3H), 4.32 (q, *J* = 7.2 Hz, 2H), 3.96 (br s, 2H), 3.51 (br d, *J* = 11.4 Hz, 2H), 3.37 (td, *J =* 7.4, 5.4 Hz, 2H), 2.97 (br d, *J =* 11.5 Hz, 2H), 2.73 (t, *J =* 7.4 Hz, 2H), 1.94 - 1.84 (m, 4H), 1.39 (t, *J =* 7.2 Hz, 3H). ESI-MS *m*/*z* 434 ([M+H]⁺). |
| **I-33** | 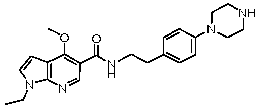 | (DMSO-*d*₆, 400 MHz) *δ:* 9.95 (br s, 1H), 8.40 (s, 1H), 8.35 (t, *J =* 5.4 Hz, 1H), 7.50 (d, *J =* 3.6 Hz, 1H), 7.10 (d, *J =* 8.5 Hz, 2H), 6.90 (d, *J =* 3.6 Hz, 1H), 6.85 (d, *J* = 8.5 Hz, 2H), 4.35 (s, 3H), 4.29 (q, *J* = 7.2 Hz, 2H), 3.37 (td, *J =* 7.4, 5.4 Hz, 2H), 3.01-2.95 (m, 4H), 2.83-2.70 (m, 6H), 1.39 (t, *J =* 7.2 Hz, 3H). ESI-MS *m*/*z* 408 ([M+H]⁺). |

### Example 2

USP28 activity was determined by the ubiquitin-rhodamine 110 (Ub-Rh110) method.

Purified USP28 and a substrate Ub-Rh110 used for determining deubiquitinase activity were purchased from R&D Systems China Co., Ltd. (R&D Systems). First, test compounds were each dissolved in DMSO to prepare a 10 mM stock solution, and then the stock solution was diluted to the desired concentration (with a DMSO content of ≤ 0.5%) with a buffer solution [containing 20 mM Tris-HCl (pH 8.0), 2 mM CaCl₂, 3 mM BME, 0.01% Prionex, and 0.01% Triton X-100]. The prepared test compound solutions at different concentrations were mixed uniformly with USP28 (final concentration: 4 nM) in a 96-well plate, and the mixture was incubated at r. t. for 30 min before the addition of Ub-Rh110 to 125 nM. The final volume of the whole reaction system was 20 µL. The released fluorescence (excitation wavelength: 485 nm, emission wavelength: 535 nm) was detected immediately on a microplate reader after the addition of the substrate. A blank control group (DMSO instead of test compound) and a substrate group (no USP28 added) were set simultaneously. The inhibitory rates of the test compounds for USP28 were calculated according to the following formula: Inhibition % = (1 - [(fluorescence valuetest compound - fluorescence valuesubstrate)/(fluorescence valuecontrol - fluorescence valuesubstrate]) × 100%

According to the inhibitory rates of the test compounds for USP28 under different concentrations, IC₅₀ values were calculated. The IC₅₀ values of the Example compounds **I-01** to **I-33** of the present disclosure for USP28 are shown in Table 2.

### Example 3

USP25 activity was determined by the Ub-Rh110 method.

Purified USP25 was purchased from R&D Systems. The experimental procedures were the same as those in Example 2. The final concentration of USP25 was 15 nM, the final concentration of the substrate Ub-Rh110 was 125 nM, and the final volume of the whole reaction system was 20 µL. The IC₅₀ values of the Example compounds **I-01** to **I-33** of the present disclosure for USP25 are shown in Table 2.

**Table 2. Inhibitory activity (IC₅₀)* of example compounds for USP28 and USP25**

| **No.** | **USP28** | **USP25** | No. | USP28 | USP25 | No. | USP28 | USP25 |
|---|---|---|---|---|---|---|---|---|
| **I-01** | + | + | **I-12** | ++++ | +++ | **I-23** | ++++ | ++++ |
| **I-02** | ++++ | ++++ | **I-13** | ++++ | ++++ | **I-24** | +++ | ++ |
| **I-03** | ++ | ++ | **I-14** | ++++ | ++++ | **I-25** | ++++ | +++ |
| **I-04** | + | | **I-15** | ++++ | ++++ | **I-26** | +++ | ++ |
| **I-05** | ++++ | +++ | **I-16** | ++++ | ++++ | **I-27** | ++++ | ++++ |
| **I-06** | +++ | ++ | **I-17** | ++++ | +++ | **I-28** | ++++ | ++++ |
| **I-07** | ++++ | ++++ | **I-18** | ++++ | +++ | **I-29** | ++++ | +++ |
| **I-08** | ++++ | +++ | **I-19** | ++++ | +++ | **I-30** | ++++ | +++ |
| **I-09** | +++ | +++ | **I-20** | ++++ | +++ | **I-31** | + | |
| **I-10** | ++ | | **I-21** | +++ | ++ | **I-32** | +++ | + |
| **I-11** | +++ | ++ | **I-22** | +++ | ++ | **I-33** | +++ | + |

| | | | | | | | | |
|---|---|---|---|---|---|---|---|---|
| * The symbols correspond to the IC₅₀ ranges as follows: ++++, < 0.05 *µ*M; +++, 0.05-0.5 *µ*M; ++, 0.5-1.0 *µ*M; +, 1.0-10.0 *µ*M; and -, > 10 *µ*M. | | | | | | | | |

### Example 4

With reference to Example 2 and Example 3, USP28 and USP25 inhibitor activity tests were performed on representative example compounds of the present disclosure and the following comparative compound structures. The test results (IC₅₀ of the comparative compound /IC₅₀ of the compound of the present disclosure) are shown in Table 3. The results further indicated that the compound structures of the present disclosure had good inhibitory activity.

**Table 3. Inhibitory activity of the compounds of the present disclosure and comparative compounds for USP28 and USP25**

| Example of the present disclosure | Comparative compound | USP28 Inhibitory activity ratio (IC₅₀ of the comparative compound /IC₅₀ of the compound of the present disclosure) | USP25 Inhibitory activity ratio (IC₅₀ of the comparative compound /IC₅₀ of the compound of the present disclosure) |
|---|---|---|---|
| 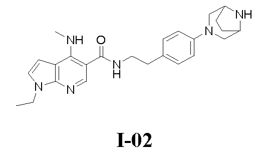 | 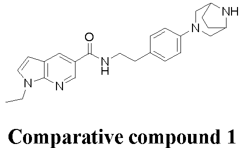 | 8.34 | 8.66 |
| 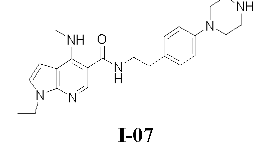 | 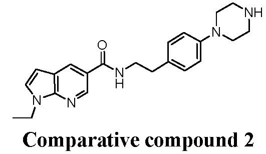 | 39.56 | 20.93 |
| | 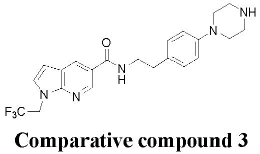 | 16.54 | 29.48 |
| 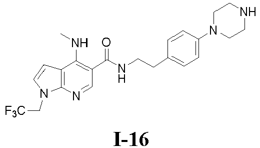 | | | |
| 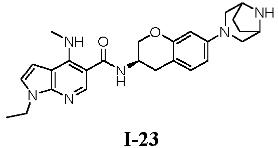 | 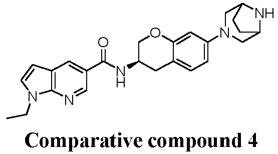 | 19.37 | 31.33 |
| | 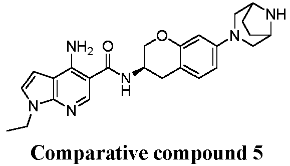 | 54.53 | 109.14 |

The embodiments of the technical solutions of the present disclosure have been described above by way of example. It should be understood that the protection scope of the present disclosure is not limited to the embodiments described above. Any modification, equivalent replacement, improvement, and the like made by those skilled in the art without departing from the spirit and principle of the present disclosure shall fall within the protection scope of the claims of the present application.

## Claims

1. A compound of formula **I**, and a racemate, a stereoisomer, a tautomer, an isotope-labeled form, a N-oxide, a solvate, a polymorph, a metabolite, an ester, a pharmaceutically acceptable salt or a prodrug thereof: 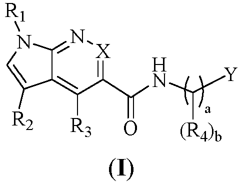 wherein:
X is selected from N or CH;
Y is selected from 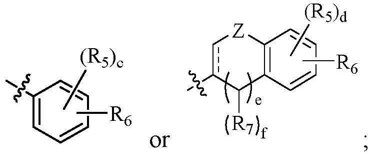
Z is selected from NR₈, O, S, or CR₉R₁₀; the dashed bond represents that there can be a bond or not;
a is selected from 0, 1, 2, 3, 4, 5, or 6;
b is selected from 1 or 2;
c is selected from 1, 2, 3, or 4;
d is selected from 1, 2, or 3;
e is selected from 0 or 1;
f is selected from 1 or 2;
R₁ is selected from hydrogen or optionally unsubstituted or substituted (C₁-C₁₂) aliphatic hydrocarbyl;
R₂ is selected from hydrogen, halogen, or optionally unsubstituted or substituted (C₁-C₁₂) aliphatic hydrocarbyl;
R₃ is selected from halogen, hydroxyl, optionally unsubstituted or substituted (C₁-C₁₂) aliphatic hydrocarbyloxy, or optionally unsubstituted or substituted (C₁-C₁₂) aliphatic hydrocarbylamino;
R₄ can each be identical or different, and are each independently selected from hydrogen, halogen, and optionally unsubstituted or substituted (C₁-C₁₂) aliphatic hydrocarbyl;
R₅ and R₇ can each be identical or different, and are each independently selected from hydrogen, halogen, hydroxyl, amino, optionally unsubstituted or substituted (C₁-C₁₂) aliphatic hydrocarbyl, and optionally unsubstituted or substituted (C₁-C₁₂) aliphatic hydrocarbyloxy;
R₆ is selected from hydrogen, halogen, hydroxyl, amino, and optionally unsubstituted or substituted (C₁-C₁₂) aliphatic hydrocarbyl, or 3- to 20-membered heterocyclyl or 5- to 20-membered heteroaryl containing one, two, or more N atoms and/or O atoms, unsubstituted or optionally substituted with one, two, or more R₁₁;
R₈, R₉, and R₁₀ are selected from hydrogen or optionally unsubstituted or substituted (C₁-C₁₂) aliphatic hydrocarbyl;
R₁₁ can each be identical or different, and are each independently selected from hydrogen, halogen, hydroxyl, amino, and optionally unsubstituted or substituted (C₁-C₁₂) aliphatic hydrocarbyl.

2. The compound as claimed in claim 1, wherein X is selected from CH;
preferably, Y is selected from 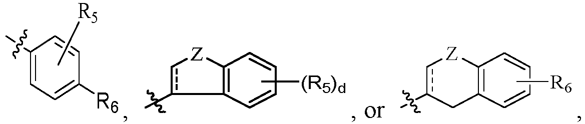 wherein Z,
R₅, R₆, and d independently have the definitions described above; for example, Y is selected from 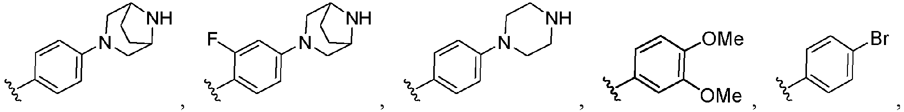 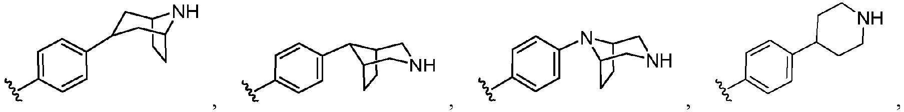 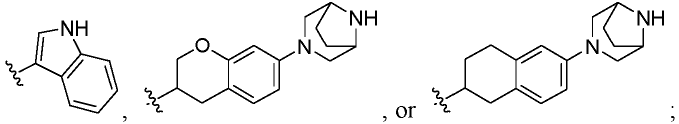
preferably, Z is selected from nitrogen-hydrogen (NH), oxygen (O), sulfur (S), or methylene (CH₂).

3. The compound as claimed in claim 1 or 2, wherein R₁ is selected from hydrogen or unsubstituted or substituted (C₁-C₆) aliphatic hydrocarbyl; for example, (C₁-C₆) aliphatic hydrocarbyl or halogenated (C₁-C₆) aliphatic hydrocarbyl;
preferably, R₁ is selected from hydrogen or optionally unsubstituted or substituted (C₁-C₆) alkyl; for example, H, (C₁-C₆) alkyl, or halogenated (C₁-C₆) alkyl;
preferably, R₁ is selected from the following groups: H, methyl, ethyl, propyl, isopropyl, butyl, isobutyl, *sec*-butyl, pentyl, isopentyl, *see*-pentyl, CF₃, CHF₂CH, CH₂FCH, CF₃CH₂, CHF₂CH₂, or CH₂FCH₂;
preferably, R₂ is selected from hydrogen or optionally unsubstituted or substituted (C₁-C₆) alkyl; for example, H, (C₁-C₆) alkyl, or halogenated (C₁-C₆) alkyl;
preferably, R₃ is selected from halogen, hydroxyl, unsubstituted or substituted (C₁-C₆) aliphatic hydrocarbylamino, or unsubstituted or substituted (C₁-C₆) aliphatic hydrocarbyloxy; for example, halogen, (C₁-C₆) alkylamino, halogenated (C₁-C₆) alkylamino, (C₁-C₆) alkyloxy, or halogenated (C₁-C₆) alkyloxy;
preferably, R₃ is selected from halogen, hydroxyl, optionally unsubstituted or substituted (C₁-C₆) alkyloxy, or optionally unsubstituted or substituted (C₁-C₆) alkylamino; for example, F, Cl, Br, I, (C₁-C₆) alkylamino, halogenated (C₁-C₆) alkylamino, (C₁-C₆) alkyloxy, or halogenated (C₁-C₆) alkyloxy; preferably, R₃ is selected from the following groups: Cl, methylamino (Me-NH), fluoromethylamino (CH₂F-NH), difluoromethylamino (CHF₂-NH), trifluoromethylamino (CF₃-NH), ethylamino (Et-NH), propylamino ("Pr-NH), isopropylamino (*ⁱ*Pr-NH), or methoxy (CH₃O);
preferably, R₄ is selected from hydrogen or optionally unsubstituted or substituted (C₁-C₆) alkyl; for example, H, (C₁-C₆) alkyl, or halogenated (C₁-C₆) alkyl; such as H or methyl;
preferably, R₅ is selected from hydrogen, halogen, optionally unsubstituted or substituted (C₁-C₆) alkyl, or optionally unsubstituted or substituted (C₁-C₆) alkoxy; for example, H, F, Cl, Br, I, (C₁-C₆) alkyl, (C₁-C₆) alkoxy; such as H, F, Br, methyl, or methoxy.

4. The compound as claimed in any one of claims 1-3, wherein R₆ is selected from hydrogen, halogen, hydroxyl, amino, and optionally unsubstituted or substituted (C₁-C₆) aliphatic hydrocarbyl, or 3- to 14-membered heterocyclyl or 5- to 14-membered heteroaryl containing one, two, or more N atoms and/or O atoms, unsubstituted or optionally substituted with one, two, or more R₁₁; R₁₁ are each identical or different, and are each independently selected from hydrogen, halogen, hydroxyl, amino, and optionally unsubstituted or substituted (C₁-C₆) aliphatic hydrocarbyl;
preferably, R₆ is selected from 3- to 10-membered heterocyclyl containing one or two N as heteroatoms, unsubstituted or optionally substituted with one, two, or more R₁₁; R₁₁ are each identical or different, and are each independently selected from hydrogen or (C₁-C₆) alkyl;
preferably, R₆ is selected from 6- to 8-membered heterocyclyl containing one or two N atoms and/or O atoms; for example, the following groups:

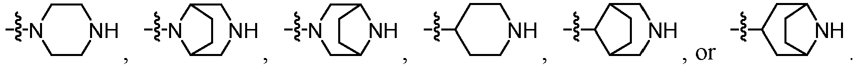

5. The compound as claimed in any one of claims 1-4, wherein R₇, R₈, R₉, and R₁₀ are identical or different, and are each independently selected from hydrogen or optionally unsubstituted or substituted (C₁-C₆) alkyl; for example, H, (C₁-C₆) alkyl, or halogenated (C₁-C₆) alkyl; such as H or methyl.

6. The compound as claimed in any one of claims 1-5, wherein the compound of formula I is selected from structures **II-a** or **II-b:** 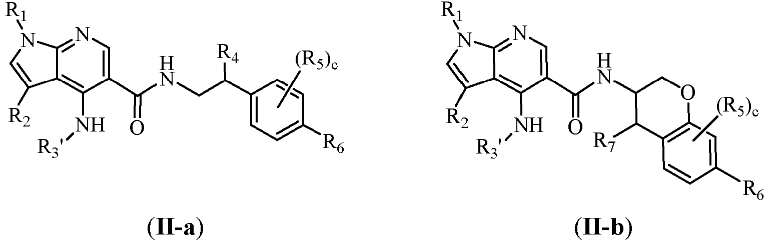 R₂, R₄, R₅, R₇, and c described in formula **II-a** and formula **II-b** have the definitions described in any one of claims 1-5; R₃' is selected from optional (C₁-C₆) aliphatic hydrocarbyl or (C₁-C₆) aliphatic hydrocarbyl comprising one, two, or more halogen and/or hydroxyl substitutions; for example, R₃' can be methyl, fluoromethyl, difluoromethyl, trifluoromethyl, ethyl, propyl, and isopropyl.

7. The compound as claimed in any one of claims 1-6, wherein the compound represented by formula **I** is selected from the following compounds:

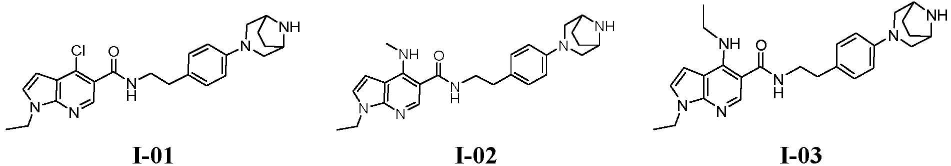

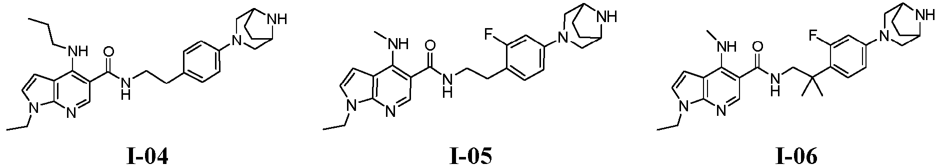

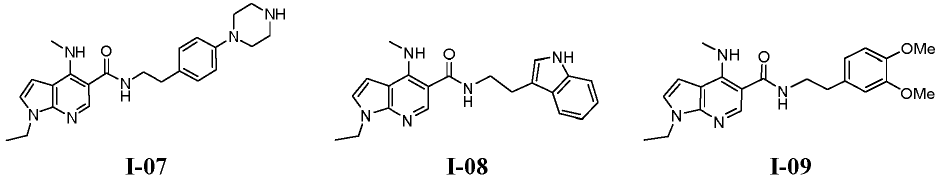

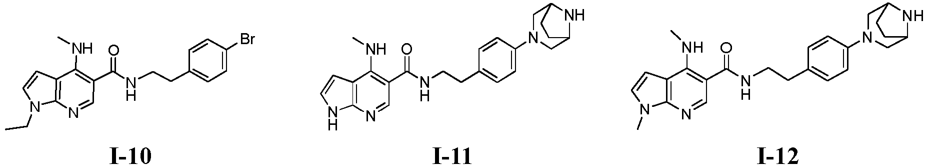

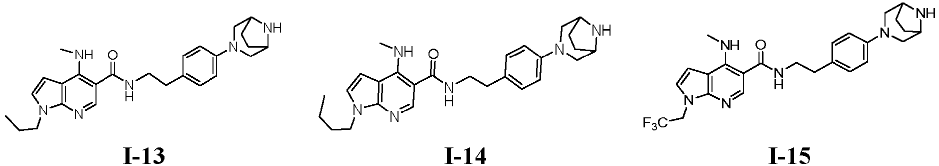

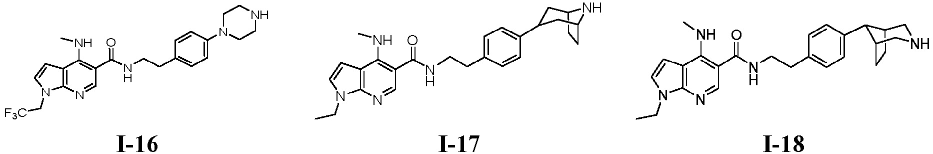

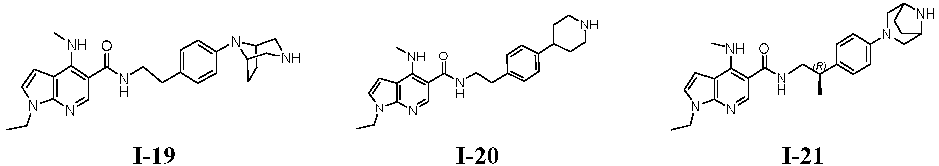

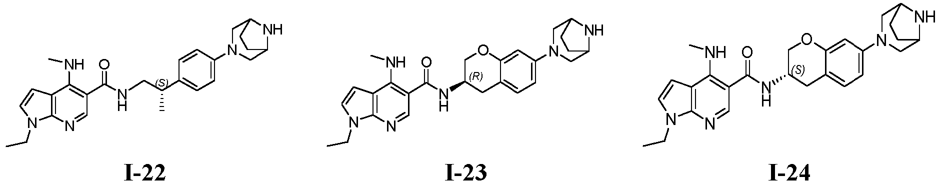

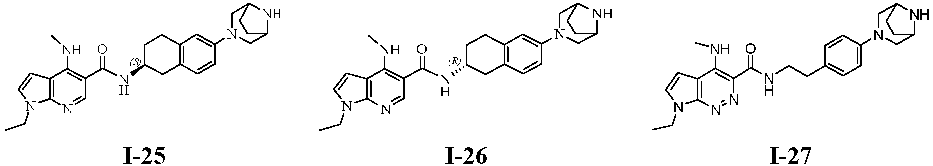

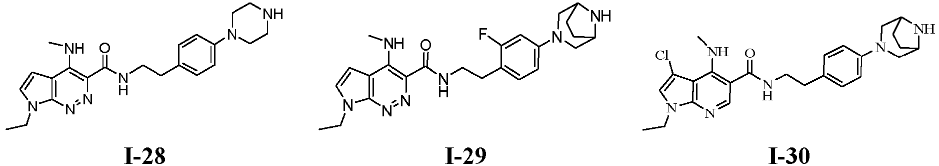

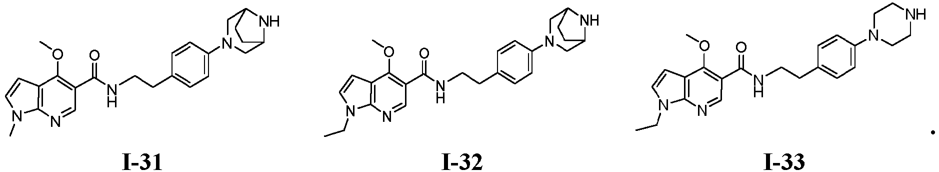

8. A pharmaceutical composition, comprising the compound represented by formula **I**, and the racemate, the stereoisomer, the tautomer, the isotope-labeled form, the *N*-oxide, the solvate, the polymorph, the metabolite, the ester, the pharmaceutically acceptable salt or the prodrug thereof as claimed in any one of claims 1-7.

9. Use of the compound represented by formula **I**, and the racemate, the stereoisomer, the tautomer, the isotope-labeled form, the *N*-oxide, the solvate, the polymorph, the metabolite, the ester, the pharmaceutically acceptable salt or the prodrug thereof as claimed in any one of claims 1-7, or the pharmaceutical composition as claimed in claim 8 for manufacturing a medicament for the treatment of diseases or disorders related to the inhibition of USP28 and/or USP25.

10. The use as claimed in claim 9, wherein the diseases or disorders related to USP28 and/or USP25 include cancer, inflammation, autoimmune diseases, viral infection, and bacterial infection.
